# EUROPEAN PATENT APPLICATION

(11) **EP 1 900 739 A1**
(43) Date of publication of application: **19.03.2008**
(21) Application number: 06119832.1
(22) Date of filing: 30.08.2006
(51) Int. Cl.: C07D 487/04, A61K 31/5025, A61K 31/519, A61P 3/10, A61P 7/12, A61P 11/08, A61P 17/06, A61P 19/02, A61P 29/00, A61P 37/02, A61P 37/08

(54) **Diazolodiazine derivatives as kinase inhibitors**

(71) Applicant: CELLZOME AG, 69117 Heidelberg (DE)
(72) Inventor: Wilson, Francis, Hertfordshire AL7 4QJ (GB); Ramsden, Nigel, Hertfordshire SG8 7QP (GB)
(74) Representative: Huhn, Michael

(57) **Abstract**

The invention relates to compounds of formula (I) 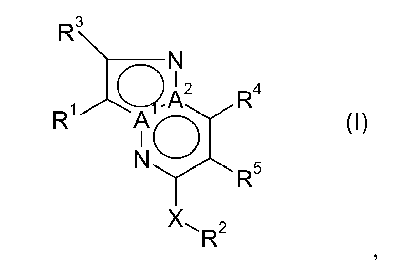 wherein A¹, A², X and R¹ to R⁵ have the meaning as cited in the description and the claims. Said compounds are useful as Itk protein kinase inhibitors for the treatment or prophylaxis of immunological, inflammatory or allergic disorders. The invention also relates to pharmaceutical compositions including said compounds, the preparation of such compounds as well as the production of and use as medicaments.

## Description

The present invention relates to a novel class of kinase inhibitors, including pharmaceutically acceptable salts, prodrugs and metabolites thereof, which are useful for modulating protein kinase activity for modulating cellular activities such as signal transduction, proliferation, differentiation, programmed cell death, migration and cytokine secretion. More specifically the invention provides compounds which inhibit, regulate and/or modulate kinase activity, in particular Itk activity, and signal transduction pathways relating to cellular activities as mentioned above. Furthermore, the present invention relates to pharmaceutical compositions comprising said compounds, e.g. for the treatment of diseases such as immunological, inflammatory and allergic disorders, and processes for preparing said compounds.

Protein kinases participate in the signaling events which control the activation, growth, differentiation and survival of cells in response to extracellular mediators or stimuli such as growth factors, cytokines or chemokines. In general, these kinases are classified in two groups, those that preferentially phosphorylate tyrosine residues and those that preferentially phosphorylate serine and/or threonine residues. The tyrosine kinases include membrane-spanning growth factor receptors such as the epidermal growth factor receptor (EGFR) and cytosolic non-receptor kinases such as Src family kinases (Lck and Lyn), the Syk family kinases (ZAP-70 and Syk) and the Tec family kinases (e.g. Itk).

Inappropriately high protein kinase activity is involved in many diseases including cancer, metabolic diseases, immunological diseases and inflammatory disorders. This can be caused either directly or indirectly by the failure of control mechanisms due to mutation, overexpression or inappropriate activation of the enzyme. In all of these instances, selective inhibition of the kinase is expected to have a beneficial effect.

Protein tyrosine kinases - both receptor tyrosine kinases and non-receptor kinases - are essential for the activation and proliferation of cells of the immune system. Among the earliest detectable events upon the immunoreceptor activation in mast cells, T cells and B cells is the stimulation of non-receptor tyrosine kinases. Immune receptors such as the high-affinity IgE receptor (FcεRI), T cell antigen receptor (TCR) and B cell receptor (BCR), consist of antigen-binding subunits and signal transducing subunits. The signal transducing chain contains one or more copies of immunoreceptor tyrosine-based activation motifs (ITAMSs). For TCR activation, ITAMS located in the CD3 molecule are phosphorylated by Lck and Fyn, two Src family tyrosine kinases, followed by recruitment and activation of ZAP-70, a member of the Syk family of tyrosine kinases. These activated tyrosine kinases then phosphorylate downstream adaptor molecules such as LAT (linker for activation of T cells) and SLP-76 (SH2 domain-containing leukocyte protein of 76 kDa). This step leads to the activation of multiple downstream signaling molecules such as inducible T cell kinase (Itk), PLCγ1 and PI3 kinase (Wong, 2005, Current Opinion in Pharmacology 5, 1-8).

The Tec family now comprises five members (Tec, Btk, Itk, Rlk and Bmx) which are expressed mainly by hematopoietic cells and play a central role in signaling through immune receptors such as the high-affinity IgE receptor (FcεRI), T cell antigen receptor (TCR) and B cell receptor (BCR) (Smith et al., 2001, Bioessays 23, 436-446). The members of the Tec family share a common protein domain organization. They have an amino-terminal Pleckstrin Homology domain, a Tec homology domain with one or two proline-rich regions, Src homology 3 (SH3) and 2 (SH2) protein interaction domains and a carboxy-terminal kinase domain. Activation of the Tec family kinases requires several steps: recruitment to the plasma membrane through their Pleckstrin Homology domain, phosphorylation by Src family kinases and interactions with proteins that bring them into the vicinity of immune receptor signaling complexes (Schwartzberg et al., 2005, Nature Reviews Immunology 5, 284-295).

Tec family kinases are essential for B cell development and activation. Patients with mutated Btk display a block in B cell development resulting in the almost complete absence of B cells and plasma cells, reduced Ig levels and an impaired humoral immune response (Smith et al., 2001, Bioassays 23, 436-446).

In addition, Tec kinases play a role in mast cell activation through the high-affinity IgE receptor (FcεRI). Itk and Btk are expressed in mast cells and are activated by FcεRI crosslinking (Hata et al., 1998, J. Biol. Chem. 273, 19979-10987). Both acute and late phase inflammatory allergic responses are significantly reduced in Itk-deficient mice when challenged with allergen via the airways. Importantly, airway mast cell degranulation is impaired despite wild-type levels of allergen-specific IgE and IgG1 (Forssell et al., 2005, Am. J. Respir. Cell Mol. Bio. 32, 511-520).

T cells express three Tec kinases (Itk, Rlk and Tec) which are involved in T cell receptor (TCR) signaling (Berg et al., 2005, Ann. Rev. Immunol. 23, 549-600). The study of genetically manipulated mice in which the gene encoding the Itk protein is deleted gives important information about the physiological and pathophysiological function of Itk. Itk-deficient (Itk^{-/-}) mice display a calcium mobilization defect after TCR stimulation (Liu et al, 1998, J. Exp. Med. 187, 1721-1727). In addition, Itk^{-/-} mice have specific defects in T Helper 2 (T_{H}2) cell development (Fowell et al., 1999, Immunity 11, 399-409; Schaeffer et al., 1999, Science 284, 638-641). T_{H}2-cell responses play a role in the pathology of allergic asthma characterized by an increased number of T_{H}2 cells in the lungs, increased T_{H}2 cytokine secretion and mucus production. In a mouse model of allergic asthma, Itk-deficient mice show decreased interleukin 5 (IL-5) and interleukin 13 (IL-13) secretion, less mucus production and reduced T cell infiltration in the lungs (Mueller and August, 2003, J. Immunol. 170, 5056-5063). This study suggests that Itk is important for the pathology of allergic asthma and suggests that Itk is a potential therapeutic target for asthma. This notion is further corroborated by studies with compounds that selectively inhibit Itk kinase activity (Lin et al., 2004, Biochemistry 43, 11056-11062).

By contrast, Itk expression is elevated in T cells from patients with Atopic Dermatitis, a T_{H}2 cell mediated disease (Matsumoto et al., 2002, Int. Archiv. Allergy Immunol 129, 327-340). Taken together, these reports suggest that Itk is a suitable therapeutic target for immunological, inflammatory and allergic disorders provided that inhibitors with sufficient potency and selectivity can be identified.

Thus, an object of the present invention is to provide a new class of compounds as kinase inhibitors, especially as Itk inhibitors, which may be effective in the treatment or prophylaxis of immunological, inflammatory, allergic disorders or other diseases or disorders associated with Itk kinase.

Accordingly, the present invention provides compounds of formula (I) 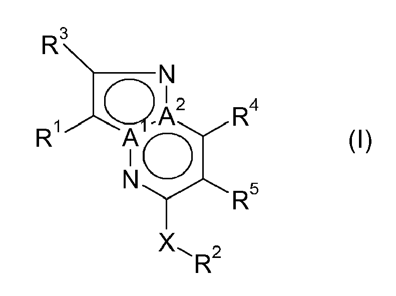 or a pharmaceutically acceptable salt, prodrug or metabolite thereof, wherein
one of A¹, A² is N and the other is C;
X is O; S or NR⁶;
R¹ is T¹;
R² is T²; C₁₋₆ alkyl; C(O)OR⁷; C(O)R⁷; C(O)N(R⁷R^{7a}); S(O)₂N(R⁷R^{7a}); S(O)N(R⁷R^{7a}); S(O)₂R⁷; or S(O)R⁷, wherein C₁₋₆ alkyl is optionally substituted with one or more R⁸;
R³, R⁴, R⁵ are independently selected from the group consisting of H; halogen; CN; C(O)OR⁹; OR⁹; C(O)R⁹; C(O)N(R⁹R^{9a}); S(O)₂N(R⁹R^{9a}); S(O)N(R⁹R^{9a}); S(O)₂R⁹; S(O)R⁹; N(R⁹)S(O)₂N(R^{9a}R^{9b}); SR⁹; N(R⁹R^{9a}); OC(O)R⁹; N(R⁹)C(O)R^{9a}; N(R⁹)S(O)₂R^{9a}; N(R⁹)S(O)R^{9a}; N(R⁹)C(O)N(R^{9a}R^{9b}); N(R⁹)C(O)OR^{9a}; OC(O)N(R⁹R^{9a}); and C₁₋₆ alkyl, wherein C₁₋₆ alkyl is optionally substituted with one or more halogen, which are the same or different;
R⁶, R^{7a}, R⁹, R^{9a}, R^{9b} are independently selected from the group consisting of H; and C₁₋₆ alkyl, wherein C₁₋₆ alkyl is optionally substituted with one or more halogen, which are the same or different;
R⁷ is T²; or C₁₋₆ alkyl, wherein C₁₋₆ alkyl is optionally substituted with one or more R⁸;
T¹ is C₃₋₇ cycloalkyl; heterocyclyl; heterobicyclyl; phenyl; naphthyl; indenyl; indanyl; tetralinyl; decalinyl; or adamantyl, wherein T¹ is optionally substituted with one or more R¹⁰;
T² is C₃₋₇ cycloalkyl; heterocyclyl; heterobicyclyl; phenyl; naphthyl; indenyl; indanyl; tetralinyl; decalinyl; or adamantyl, wherein T² is optionally substituted with one or more R¹¹;
R⁸ is T²; C₁₋₆ alkyl; halogen; CN; C(O)OR¹²; OR¹²; C(O)R¹²; C(O)N(R¹²R^{12a}); S(O)₂N(R¹²R^{12a}); S(O)N(R¹²R^{12a}); S(O)₂R¹²; S(O)R¹² ; N(R¹²)S(O)₂N(R^{12a}R^{12b}); SR¹²; N(R¹²R^{12a}); OC(O)R¹²; N(R¹²)C(O)R^{12a}; N(R¹²)S(O)₂R^{12a}; N(R¹²)S(O)R^{12a}; N(R¹²)C(O)N(R^{12a}R^{12b}); N(R¹²)C(O)OR^{12a}; or OC(O)N(R¹²R^{12a}), wherein C₁₋₆ alkyl is optionally substituted with one or more R¹³;
R¹⁰, R¹¹ are independently selected from the group consisting of T³; C₁₋₆ alkyl; halogen; CN; C(O)OR¹²; OR¹²; oxo (=O), where the ring is at least partially saturated; C(O)R¹²; C(O)N(R¹²R^{12a}); S(O)₂N(R¹²R^{12a}); S(O)N(R¹²R^{12a}); S(O)₂R¹²; S(O)R¹²; N(R¹²)S(O)₂N(R^{12a}R^{12b}); SR¹² ; N(R¹²R^{12a}); OC(O)R¹²; N(R¹²)C(O)R^{12a}; N(R¹²)S(O)₂R^{12a}; N(R¹²)S(O)R^{12a}; N(R¹²)C(O)N(R^{12a}R^{12b}); N(R¹²)C(O)OR^{12a}; and OC(O)N(R¹²R^{12a}), wherein C₁₋₆ alkyl is optionally substituted with one or more R¹³;
R¹², R^{12a}, R^{12b} are independently selected from the group consisting of H; T³; and C₁₋₆ alkyl, wherein C₁₋₆ alkyl is optionally substituted with one or more R¹⁴;
T³ is C₃₋₇ cycloalkyl; heterocyclyl; heterobicyclyl; phenyl; naphthyl; indenyl; indanyl; tetralinyl; decalinyl; or adamantyl, wherein T³ is optionally substituted with one or more R¹⁵;
R¹³, R¹⁴ are independently selected from the group consisting of halogen; CN; C(O)OR¹⁶; OR¹⁶ ; C(O)R¹⁶; C(O)N(R¹⁶R^{16a}); S(O)₂N(R¹⁶R^{16a}); S(O)N(R¹⁶R^{16a}); S(O)₂R¹⁶; S(O)R¹⁶; N(R¹⁶)S(O)₂N(R^{16a}R^{16b}); SR¹⁶; N(R¹⁶R^{16a}); OC(O)R¹⁶; N(R¹⁶)C(O)R^{16a}; N(R¹⁶)S(O)₂R^{16a}; N(R¹⁶)S(O)R^{16a}; N(R¹⁶)C(O)N(R^{16a}R^{16b}); N(R¹⁶)C(O)OR^{16a}; OC(O)N(R¹⁶R^{16a}); and C₁₋₆ alkyl, wherein C₁₋₆ alkyl is optionally substituted with one or more halogen, which are the same or different;
R¹⁵ is halogen; CN; C(O)OR¹⁶; OR¹⁶; oxo (=O), where the ring is at least partially saturated; C(O)R¹⁶; C(O)N(R¹⁶R^{16a}); S(O)₂N(R¹⁶R^{16a}); S(O)N(R¹⁶R^{16a}); S(O)₂R¹⁶; S(O)R¹⁶; N(R¹⁶)S(O)₂N(R^{16a}R^{16b}); SR¹⁶; N(R¹⁶R^{16a}); OC(O)R¹⁶; N(R¹⁶)C(O)R^{16a}; N(R¹⁶)S(O)₂R^{16a}; N(R¹⁶)S(O)R^{16a}; N(R¹⁶)C(O)N(R^{16a}R^{16b}); N(R¹⁶)C(O)OR^{16a}; OC(O)N(R¹⁶R^{16a}); or C₁₋₆ alkyl, wherein C₁₋₆ alkyl is optionally substituted with one or more halogen, which are the same or different;
R¹⁶, R^{16a}, R^{16b} are independently selected from the group consisting of H; and C₁₋₆ alkyl, wherein C₁₋₆ alkyl is optionally substituted with one or more halogen, which are the same or different.

In case a variable or substituent can be selected from a group of different variants and such variable or substituent occurs more than once the respective variants can be the same or different.

Within the meaning of the present invention the terms are used as follows:
"Alkyl" means a straight-chain or branched carbon chain that may contain double or triple bonds. It is generally preferred that alkyl doesn't contain double or triple bonds. Thus, the term "alkyl" includes within the meaning of the present invention alkyl groups as well as alkenyl and alkinyl groups. Each hydrogen of an alkyl carbon may be replaced by a substituent.
"C₁₋₄ alkyl" means an alkyl chain having 1-4 carbon atoms, e.g. if present at the end of a molecule: methyl, ethyl, -CH=CH₂, -C≡CH, n-propyl, isopropyl, -CH=CH-CH₃, -CH₂-CH=CH₂, n-butyl, isobutyl, -CH=CH-CH₂-CH₃, -CH=CH-CH=CH₂, sec-butyl tert-butyl, or e.g. -CH₂-, -CH₂-CH₂-, -CH=CH-, -CH(CH₃)-, -C(CH₂)-, -CH₂-CH₂-CH₂-, -CH(C₂H₅)-, - CH(CH₃)₂-, when two moieties of a molecule are linked by the alkyl group. Preferably, C₁₋₄ alkyl includes methyl, ethyl, n-propyl, isopropyl, n-butyl, isobutyl, sec-butyl and tert-butyl. Each hydrogen of a C₁₋₄ alkyl carbon may be replaced by a substituent.
"C₁₋₆ alkyl" means an alkyl chain having 1 - 6 carbon atoms, e.g. if present at the end of a molecule: C₁₋₄ alkyl, methyl, ethyl, -CH=CH₂, -C≡CH, n-propyl, isopropyl, -CH=CH-CH₃, - CH₂-CH=CH₂, n-butyl, isobutyl, -CH=CH-CH₂-CH₃, -CH=CH-CH=CH₂, sec-butyl; tert-butyl, n-pentyl, n-hexyl, or e.g. -CH₂-, -CH₂-CH₂-, -CH=CH-, -CH(CH₃)-, -C(CH₂)-, -CH₂-CH₂-CH₂-, -CH(C₂H₅)-, -CH(CH₃)₂-, when two moieties of a molecule are linked by the alkyl group. Preferably, C₁₋₆ alkyl includes methyl, ethyl, n-propyl, isopropyl, n-butyl, isobutyl, sec-butyl and tert-butyl, n-pentyl, and n-hexyl. Each hydrogen of a C₁₋₆ alkyl carbon may be replaced by a substituent.
"C₃₋₇ cycloalkyl" or "C₃₋₇ cycloalkyl ring" means a cyclic alkyl chain having 3 - 7 carbon atoms, e.g. cyclopropyl, cyclobutyl, cyclopentyl, cyclohexyl, cyclohexenyl, cycloheptyl. Preferably, C₃₋₇ cycloalkyl includes cyclopropyl, cyclobutyl, cyclopentyl, cyclohexyl, and cycloheptyl. Each hydrogen of a cycloalkyl carbon may be replaced by a substituent.
"Halogen" means fluoro, chloro, bromo or iodo. It is generally preferred that halogen is fluoro or chloro.
"Heterocyclyl" or "heterocycle" means a cyclopentane, cyclohexane or cycloheptane ring, preferably a cyclopentane or cyclohexane ring, that may contain up to the maximum number of double bonds (aromatic or non-aromatic ring which is fully, partially or un-saturated) wherein at least one carbon atom up to 4 carbon atoms are replaced by a heteroatom selected from the group consisting of sulfur (including -S(O)-, -S(O)₂-), oxygen and nitrogen (including =N(O)-) and wherein the ring is linked to the rest of the molecule via a carbon or nitrogen atom. Examples for heterocycles are furan, thiophene, pyrrole, pyrroline, imidazole, imidazoline, pyrazole, pyrazoline, oxazole, oxazoline, isoxazole, isoxazoline, thiazole, thiazoline, isothiazole, isothiazoline, thiadiazole, thiadiazoline, tetrahydrofuran, tetrahydrothiophene, pyrrolidine, imidazolidine, pyrazolidine, oxazolidine, isoxazolidine, thiazolidine, isothiazolidine, thiadiazolidine, sulfolane, pyran, dihydropyran, tetrahydropyran, imidazolidine, pyridine, pyridazine, pyrazine, pyrimidine, piperazine, piperidine, morpholine, tetrazole, triazole, triazolidine, tetrazolidine, azepine and homopiperazine. "Heterocycle" means also azetidine.
"Aromatic heterocyclyl" or "aromatic heterocycle" means a cyclopentane, cyclohexane or cycloheptane ring, preferably a cyclopentane or cyclohexane ring, that contains up to the maximum number of conjugated ring double bonds. Examples for aromatic heterocycles are furan, thiophene, pyrrole, imidazole, pyrazole, oxazole, isoxazole, thiazole, isothiazole, thiadiazole, pyranium, pyridine, pyridazine, pyrazine, pyrimidine, piperazine, triazole, and tetrazole.
"Non-aromatic heterocyclyl" or "non-aromatic heterocycle" means heterocyclyl or heterocycle other than an aromatic heterocyclyl or aromatic heterocycle, especially a fully saturated heterocyclyl or heterocycle.
"Heterobicyclyl" or "heterobicycle" means a heterocycle which is condensed with phenyl, C₃₋₇ cycloalkyl or an additional heterocycle to form a bicyclic ring system. "Condensed" to form a bicyclic ring means that two rings are attached to each other by sharing two ring atoms. Examples for heterobicycles are indole, indoline, benzofuran, benzothiophene, benzoxazole, benzisoxazole, benzothiazole, benzisothiazole, benzimidazole, benzimidazoline, benzotriazole, [1,24]triazolo[1,5a]pyridine, quinoline, quinazoline, dihydroquinazoline, quinoline, dihydroquinoline, tetrahydroquinoline, decahydroquinoline, isoquinoline, decahydroisoquinoline, tetrahydroisoquinoline, dihydroisoquinoline, benzazepine, imidazopyridazine, pyrazolopyrimidine, purine and pteridine.
"Aromatic heterobicyclyl" or "aromatic heterobicycle" means an aromatic heterocycle which is condensed with phenyl or an additional aromatic heterocycle to form a bicyclic ring system. "Condensed" to form a bicyclic ring means that two rings are attached to each other by sharing two ring atoms. Examples for aromatic heterobicycles are indole, benzofuran, benzothiophene, benzoxazole, benzisoxazole, benzothiazole, benzisothiazole, benzimidazole, benzotriazole, [1,24]triazolo[1,5a]pyridine, quinoline, isoquinoline, imidazopyridazine, pyrazolopyrimidine, purine and pteridine.
"Non-aromatic heterobicyclyl" or "non-aromatic heterobicycle" means heterobicyclyl or heterobicycle other than an aromatic heterobicyclyl or aromatic heterobicycle.

Preferred compounds of formula (I) are those compounds in which one or more of the residues contained therein have the meanings given below, with all combinations of preferred substituent definitions being a subject of the present invention. With respect to all preferred compounds of the formulae (I) the present invention also includes all tautomeric and stereoisomeric forms and mixtures thereof in all ratios, and their pharmaceutically acceptable salts.

In preferred embodiments of the present invention, the substituents mentioned below independently have the following meaning. Hence, one or more of these substituents can have the preferred or more preferred meanings given below.

Preferred compounds of the present invention are those of formula (Ia) or (Ib) 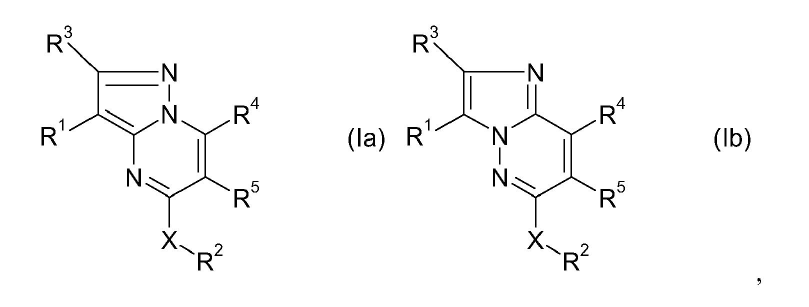 wherein X, R¹ to R⁵ have the meaning as indicated above.

Preferably, X is N(R⁶), wherein R⁶ has the meaning as indicated above.

Preferably, R¹ is T¹ and T¹ is heterocyclyl; phenyl; or heterobicyclyl. More preferred T¹ is unsubstituted aromatic heterocyclyl; substituted aromatic heterocyclyl; substituted phenyl; unsubstituted heterobicyclyl; or substituted heterobicyclyl. Even more preferred T¹ is substituted thienyl; unsubstituted thienyl; substituted benzodioxolyl; unsubstituted benzodioxolyl; substituted pyridyl; unsubstituted pyridyl; substituted pyrazolyl; unsubstituted pyrazolyl; substituted indolyl; unsubstituted indolyl; substituted pyrimidyl; unsubstituted pyrimidyl; substituted benzothienyl; unsubstituted benzothienyl; substituted benzofuranyl; unsubstituted benzofuranyl; or substituted phenyl.

Preferably, T¹ is unsubstituted or substituted with up to three R¹⁰, which are the same or different.

Preferably, R¹⁰ is C(O)R¹²; C(O)OR¹²; OR¹²; N(R¹²R^{12a}); C(O)N(R¹²R^{12a}); N(R¹²)C(O)R^{12a}; CN; N(R¹²)S(O)₂R^{12a}; T³; C₁₋₆ alkyl; C₁₋₆ alkyl substituted with up to three R¹³, which are the same or different; or halogen. More preferred R¹⁰ is C(O)CH₃; C(O)OH; OH; OCH₃; OCF₃; CH₂OH; C(O)NH₂; C(O)NHCH₂CH₂N(CH₃)₂; NHC(O)CH₃; NHS(O)₂CH₃; CH₃; NH₂; NHCH₃; N(CH₃)₂; (CH₃)₂NCH₂CH₂NH; CH=CHC(O)OH; F; or Cl.

Preferably, R¹², R^{12a} are independently selected from the group consisting of H; unsubstituted C₁₋₆ alkyl; C₁₋₆ alkyl substituted with up to three R¹³, which are the same or different; and T³.

Preferably, R¹³ is OH; OCH₃; F; NH₂; NHCH₃; N(CH₃)₂; C(O)OH; or C(O)OCH₃.

Preferably, R² is T²; C₁₋₆ alkyl; or C₁₋₆ alkyl substituted with up to three R⁸. More preferred R² is C₁₋₆ alkyl substituted with one R⁸. Even more preferred, R² is [C(R¹⁷R¹⁷ₐ)]ₙCHR⁸, wherein n is 0, 1 or 2 and wherein R¹⁷, R^{17a} are independently selected from the group consisting of H; CH₃; CH₂CH₃; CH₂CH₂CH₃ and CH₃CHCH₃, provided that the total number of carbon atoms for the C(R¹⁷R^{17a}) group or groups does not exceed 5.

Preferably, R⁸ is T²; OR¹²; or N(R¹²R^{12a}). More preferred R⁸ is T²; OH; OCH₃; NH₂; NHCH₃; or N(CH₃)₂.

Preferably, T² is substituted heterocyclyl; unsubstituted heterocyclyl; substituted C₃₋₇ cycloalkyl; unsubstituted phenyl; substituted phenyl; substituted heterobicyclyl; or unsubstituted heterobicyclyl. More preferred T² is substituted pyridyl; unsubstituted pyridyl; substituted cyclohexyl; unsubstituted phenyl; substituted phenyl; substituted indolyl; unsubstituted indolyl; substituted pyranyl; unsubstituted pyranyl; substituted thienyl; unsubstituted thienyl; substituted furyl; unsubstituted furyl; substituted benzothienyl; unsubstituted benzothienyl; substituted morpholinyl; or unsubstituted morpholinyl.

Preferably, R¹¹ is N(R¹²)C(O)R^{12a}; OR¹²; halogen; C₁₋₆ alkyl; N(R¹²)S(O)₂R^{12a}; or T³. More preferred R¹¹ is NHC(O)CH₃; OH; OCH₃; F; Cl; CH₃; CH₂CH₃; NHS(O)₂CH₃; or T³.

Preferably, R¹², R^{12a} are independently selected from the group consisting of H; and C₁₋₆ alkyl.

Preferably, T³ is heterocyclyl; or phenyl.

Preferably, R³; R⁴; R⁵ are independently selected from the group consisting of H; F; Cl; C(O)OH; C(O)OCH₃; OH; OCH₃; C(O)CH₃; C(O)NH₂; C(O)NHCH₃; C(O)N(CH₃)₂; NH₂;

NHCH₃; N(CH₃)₂; and CH₃. More preferred R³; R⁴; R⁵ are H.

Compounds of formula (I) in which some or all of the above-mentioned groups have the preferred or more preferred meanings are also an object of the present invention.

Preferred compounds of the present invention are those which are selected from the group consisting of
1-{5-[6-(3-Hydroxy-propylamino)-imidazo[1,2-b]pyridazin-3-yl]-thiophen-2-yl}-ethanone;
4-[6-(2-Methoxy-ethylamino)-imidazo[1,2-b]pyridazin-3-yl]-phenol;
4-[6-(Tetrahydro-pyran-4-ylamino)-imidazo[1,2-b]pyridazin-3-yl]-phenol;
(2-Methoxy-ethyl)-[3-(5-methoxy-pyridin-3-yl)-imidazo[1,2-b]pyridazin-6-yl]-amine;
4-[6-(3-Hydroxy-propylamino)-imidazo[1,2-b]pyridazin-3-yl]-benzonitrile;
{4-[6-(Tetrahydro-pyran-4-ylamino)-imidazo[1,2-b]pyridazin-3-yl]-phenyl}-methanol;
{3-[6-(Tetrahydro-pyran-4-ylamino)-imidazo[1,2-b]pyridazin-3-yl]-phenyl}-methanol;
3-(3-Furan-3-yl-imidazo[1,2-b]pyridazin-6-ylamino)-propan-1-ol;
2-Methoxy-4-[6-(tetrahydro-pyran-4-ylamino)-imidazo[1,2-b]pyridazin-3-yl]-phenol;
3-[6-(1-Hydroxymethyl-propylamino)-imidazo [1,2-b]pyridazin-3-yl]-benzamide;
N-{3-[6-(1-Hydroxymethyl-propylamino)-imidazo[1,2-b]pyridazin-3-yl]-phenyl}-methanesulfonamide;
[3-(4-Morpholin-4-yl-phenyl)-imidazo[1,2-b]pyridazin-6-yl]-(2-pyridin-2-yl-ethyl)-amine;
(3-Pyridin-4-yl-imidazo[1,2-b]pyridazin-6-yl)-thiophen-2-ylmethyl-amine;
1-(5-{6-[(Pyridin-2-ylmethyl)-amino]-imidazo[1,2-b]pyridazin-3-yl}-thiophen-2-yl)-ethanone;
Furan-2-ylmethyl-(3-pyridin-3-yl-imidazo[1,2-b]pyridazin-6-yl)-amine;
(3-Pyridin-3-yl-imidazo[1,2-b]pyridazin-6-yl)-thiophen-2-ylmethyl-amine;
Furan-2-ylmethyl-(3-pyridin-4-yl-imidazo[1,2-b]pyridazin-6-yl)-amine;
1-(5-{6-[(Thiophen-2-ylmethyl)-amino]-imidazo[1,2-b]pyridazin-3-yl}-thiophen-2-yl)-ethanone;
4-[3-(3-Trifluoromethoxy-phenyl)-imidazo[1,2-b]pyridazin-6-ylamino]-cyclohexanol;
4-[3-(1H-Indol-5-yl)-imidazo[1,2-b]pyridazin-6-ylamino]-cyclohexanol;
4-[3-(3,4-Dimethoxy-phenyl)-imidazo[1,2-b]pyridazin-6-ylamino]-cyclohexanol;
N-{3-[6-(4-Hydroxy-cyclohexylamino)-imidazo[1,2-b]pyridazin-3-yl]-phenyl}-acetamide;
(3-Pyrimidin-5-yl-imidazo[1,2-b]pyridazin-6-yl)-thiophen-2-ylmethyl-amine;
3-[6-((S)-1-Hydroxymethyl-2-methyl-propylamino)-imidazo[1,2-b]pyridazin-3-yl]-benzamide;
[3-(4-Methyl-thiophen-2-yl)-imidazo[1,2-b]pyridazin-6-yl]-pyridin-2-ylmethyl-amine;
(S)-3-Methyl-2-(3-pyrimidin-5-yl-imidazo[1,2-b]pyridazin-6-ylamino)-butan-1-ol;
4-[6-((S)-1-Hydroxymethyl-2-methyl-propylamino)-imidazo[1,2-b]pyridazin-3-yl]-2-methoxy-phenol;
4-[3-(3-Dimethylamino-phenyl)-imidazo[1,2-b]pyridazin-6-ylamino]-cyclohexanol;
Furan-2-ylmethyl-(3-pyrimidin-5-yl-imidazo[1,2-b]pyridazin-6-yl)-amine;
4-[6-(4-Hydroxy-cyclohexylamino)-imidazo[1,2-b]pyridazin-3-yl]-benzonitrile;
3-[6-(4-Hydroxy-cyclohexylamino)-imidazo[1,2-b]pyridazin-3-yl]-benzoic acid;
4-[3-(4-Methoxy-phenyl)-imidazo[1,2-b]pyridazin-6-ylamino]-cyclohexanol;
4-(3-Thiophen-3-yl-imidazo[1,2-b]pyridazin-6-ylamino)-cyclohexanol;
4-[6-(4-Hydroxy-cyclohexylamino)-imidazo[1,2-b]pyridazin-3-yl]-benzoic acid;
4-[3-(4-Hydroxymethyl-phenyl)-imidazo[1,2-b]pyridazin-6-ylamino]-cyclohexanol;
4-[6-(4-Hydroxy-cyclohexylamino)-imidazo[1,2-b]pyridazin-3-yl]-2-methoxy-phenol;
1-{5-[6-(4-Hydroxy-cyclohexylamino)-imidazo[1,2-b]pyridazin-3-yl]-thiophen-2-yl}-ethanone;
4-(6-Propylamino-imidazo[1,2-b]pyridazin-3-yl)-phenol;
N,N-Dimethyl-N'-(3-thiophen-3-yl-imidazo[1,2-b]pyridazin-6-yl)-ethane-1,2-diamine;
4-[3-(2,4-Dimethoxy-pyrimidin-5-yl)-imidazo[1,2-b]pyridazin-6-ylamino]-cyclohexanol;
N-[3-(6-Propylamino-imidazo[1,2-b]pyridazin-3-yl)-phenyl]-acetamide;
N'-(3-Benzo[b]thiophen-2-yl-imidazo[1,2-b]pyridazin-6-yl)-N,N-dimethyl-ethane-1,2-diamine;
(2,4-Dimethyl-benzyl)-(3-thiophen-3-yl-imidazo[1,2-b]pyridazin-6-yl)-amine;
1-{5-[6-(3-Morpholin-4-yl-propylamino)-imidazo[1,2-b]pyridazin-3-yl]-thiophen-2-yl}-ethanone;
N'-[3-(3,4-Dimethoxy-phenyl)-imidazo[1,2-b]pyridazin-6-yl]-N,N-dimethyl-ethane-1,2-diamine;
(3-Chloro-benzyl)-(3-pyridin-4-yl-imidazo[1,2-b]pyridazin-6-yl)-amine;
(3-Benzo[1,3]dioxol-5-yl-imidazo[1,2-b]pyridazin-6-yl)-(3-morpholin-4-yl-propyl)-amine;
4-[3-(3-Hydroxymethyl-phenyl)-imidazo[1,2-b]pyridazin-6-ylamino]-cyclohexanol;
4-(3-Phenyl-imidazo[1,2-b]pyridazin-6-ylamino)-cyclohexanol;
(3-Morpholin-4-yl-propyl)-(3-thiophen-3-yl-imidazo[1,2-b]pyridazin-6-yl)-amine;
(2,4-Dimethyl-benzyl)-(3-pyridin-4-yl-imidazo[1,2-b]pyridazin-6-yl)-amine;
4-[6-(3-Morpholin-4-yl-propylamino)-imidazo[1,2-b]pyridazin-3-yl]-phenol;
4-[3-(3-Fluoro-phenyl)-imidazo[1,2-b]pyridazin-6-ylamino]-cyclohexanol;
N'-(3-Benzofuran-2-yl-imidazo[1,2-b]pyridazin-6-yl)-N,N-dimethyl-ethane-1,2-diamine;
3-(6-Propylamino-imidazo[1,2-b]pyridazin-3-yl)-benzamide;
[4-(6-Propylamino-imidazo[1,2-b]pyridazin-3-yl)-phenyl]-methanol;
Propyl-(3-pyrimidin-5-yl-imidazo[1,2-b]pyridazin-6-yl)-amine;
3-[6-(3-Morpholin-4-yl-propylamino)-imidazo[1,2-b]pyridazin-3-yl]-phenol;
2-Methoxy-4-(6-propylamino-imidazo[1,2-b]pyridazin-3-yl)-phenol;
1-{5-[5-(2-Hydroxy-ethylamino)-pyrazolo[1,5-a]pyrimidin-3-yl]-thiophen-2-yl}-ethanone;
N-{3-[5-(2-Hydroxy-ethylamino)-pyrazolo[1,5-a]pyrimidin-3-yl]-phenyl}-acetamide;
3-[6-(2-Dimethylamino-ethylamino)-imidazo [1,2-b]pyridazin-3-yl]-benzamide;
4-[3-(3-Methoxy-phenyl)-imidazo[1,2-b]pyridazin-6-ylamino]-cyclohexanol;
2-(3-Benzo[1,3]dioxol-5-yl-pyrazolo[1,5-a]pyrimidin-5-ylamino)-ethanol;
4-{5-[(Pyridin-4-ylmethyl)-amino]-pyrazolo[1,5-a]pyrimidin-3-yl}-phenol;
4-(3-Pyridin-3-yl-pyrazolo[1,5-a]pyrimidin-5-ylamino)-cyclohexanol;
3-[5-(4-Acetylamino-phenylamino)-pyrazolo[1,5-a]pyrimidin-3-yl]-benzoic acid;
4-[3-(3,4-Dimethoxy-phenyl)-pyrazolo[1,5-a]pyrimidin-5-ylamino]-cyclohexanol;
2-[3-(3,4-Dimethoxy-phenyl)-pyrazolo[1,5-a]pyrimidin-5-ylamino]-ethanol;
4-[5-(4-Hydroxy-cyclohexylamino)-pyrazolo [1,5-a]pyrimidin-3-yl]-phenol;
(1H-Indol-5-yl)-[3-(5-methoxy-pyridin-3-yl)-pyrazolo [1,5-a]pyrimidin-5-yl]-amine;
4-[5-(2-Hydroxy-ethylamino)-pyrazolo [1,5-a]pyrimidin-3-yl]-benzonitrile;
2-[3-(3,4,5-Trimethoxy-phenyl)-pyrazolo [1,5-a]pyrimidin-5-ylamino]-ethanol;
2-Methoxy-4-[5-(3,4,5-trimethoxy-phenylamino)-pyrazolo [1,5-a]pyrimidin-3-yl]-phenol;
4-[3-(6-Methoxy-pyridin-3-yl)-pyrazolo [1,5-a]pyrimidin-5-ylamino]-phenol;
4-[5-(2-Dimethylamino-ethylamino)-pyrazolo[1,5-a]pyrimidin-3-yl]-benzoic acid;
[3-(4-Chloro-phenyl)-pyrazolo[1,5-a]pyrimidin-5-yl]-pyridin-3-ylmethyl-amine;
3-[5-(6-Methoxy-pyridin-3-ylamino)-pyrazolo[1,5-a]pyrimidin-3-yl]-phenol;
(6-Methoxy-pyridin-3-yl)-[3-(6-methoxy-pyridin-3-yl)-pyrazolo[1,5-a]pyrimidin-5-yl]-amine;
4-[3-(6-Methoxy-pyridin-3-yl)-pyrazolo[1,5-a]pyrimidin-5-ylamino]-cyclohexanol;
4-[5-(4-Hydroxy-cyclohexylamino)-pyrazolo[1,5-a]pyrimidin-3-yl]-2-methoxy-phenol;
3-{5-[(Pyridin-3-ylmethyl)-amino]-pyrazolo[1,5-a]pyrimidin-3-yl}-phenol;
(3-{5-[(Pyridin-3-ylmethyl)-amino]-pyrazolo[1,5-a]pyrimidin-3-yl}-phenyl)-methanol;
3-[5-(4-Hydroxy-cyclohexylamino)-pyrazolo[1,5-a]pyrimidin-3-yl]-phenol;
2-[3-(3-Hydroxymethyl-phenyl)-pyrazolo[1,5-a]pyrimidin-5-ylamino]-ethanol;
N'-[3-(4-Methoxy-phenyl)-pyrazolo[1,5-a]pyrimidin-5-yl]-N,N-dimethyl-ethane-1,2-diamine;
N-{5-[3-(4-Hydroxy-3-methoxy-phenyl)-pyrazolo[1,5-a]pyrimidin-5-ylamino]-2-methylphenyl}-methanesulfonamide;
(E)-3-{3-[5-(4-Hydroxy-cyclohexylamino)-pyrazolo[1,5-a]pyrimidin-3-yl]-phenyl}-acrylic acid;
2-[3-(6-Methoxy-pyridin-3-yl)-pyrazolo[1,5-a]pyrimidin-5-ylamino]-ethanol;
3-[5-(2-Dimethylamino-ethylamino)-pyrazolo[1,5-a]pyrimidin-3-yl]-phenol;
[3-(1-Methyl-1H-pyrazol-4-yl)-pyrazolo[1,5-a]pyrimidin-5-yl]-(3,4,5-trimethoxy-phenyl)-amine;
4-[3-(3-Hydroxymethyl-phenyl)-pyrazolo[1,5-a]pyrimidin-5-ylamino]-cyclohexanol;
4-[5-(2-Hydroxy-ethylamino)-pyrazolo[1,5-a]pyrimidin-3-yl]-2-methoxy-phenol;
3-[5-(3,4,5-Trimethoxy-phenylamino)-pyrazolo[1,5-a]pyrimidin-3-yl]-benzamide;
4-[3-(2-Hydroxymethyl-phenyl)-pyrazolo[1,5-a]pyrimidin-5-ylamino]-cyclohexanol;
3-[5-(4-Hydroxy-cyclohexylamino)-pyrazolo[1,5-a]pyrimidin-3-yl]-benzamide;
1-{3-[5-(4-Morpholin-4-yl-phenylamino)-pyrazolo[1,5-a]pyrimidin-3-yl]-phenyl}-ethanone;
Phenyl-(3-pyridin-4-yl-pyrazolo[1,5-a]pyrimidin-5-yl)-amine;
[3-(1-Methyl-1H-pyrazol-4-yl)-pyrazolo[1,5-a]pyrimidin-5-yl]-(4-morpholin-4-yl-phenyl)-amine;
3-{5-[(Pyridin-3-ylmethyl)-amino]-pyrazolo[1,5-a]pyrimidin-3-yl}-benzamide;
1-(5-{5-[(Pyridin-3-ylmethyl)-amino]-pyrazolo[1,5-a]pyrimidin-3-yl}-thiophen-2-yl)-ethanone;
N'-(3-Benzo [1,3]dioxol-5-yl-pyrazolo[1,5-a]pyrimidin-5-yl)-N,N-dimethyl-ethane-1,2-diamine;
N-(2-Dimethylamino-ethyl)-3-{5-[(pyridin-3-ylmethyl)-amino]-pyrazolo[1,5-a]pyrimidin-3-yl}-benzamide;
N-(2-Dimethylamino-ethyl)-4-{5-[(pyridin-3-ylmethyl)-amino]-pyrazolo[1,5-a]pyrimidin-3-yl}-benzamide;
4-{5-[(Pyridin-3-ylmethyl)-amino]-pyrazolo[1,5-a]pyrimidin-3-yl}-phenol;
N-(2-Dimethylamino-ethyl)-3-[5-(6-methoxy-pyridin-3-ylamino)-pyrazolo[1,5-a]pyrimidin-3-yl]-benzamide;
1-{5-[5-(2-Dimethylamino-ethylamino)-pyrazolo[1,5-a]pyrimidin-3-yl]-thiophen-2-yl}-ethanone;
4-[3-(3-Aminomethyl-phenyl)-pyrazolo[1,5-a]pyrimidin-5-ylamino]-phenol;
4-[3-(4-Fluoro-phenyl)-pyrazolo[1,5-a]pyrimidin-5-ylamino]-cyclohexanol;
[3-(3-Chloro-phenyl)-pyrazolo[1,5-a]pyrimidin-5-yl]-(4-morpholin-4-yl-phenyl)-amine;
[3-(3-Chloro-phenyl)-pyrazolo[1,5-a]pyrimidin-5-yl]-pyridin-3-ylmethyl-amine;
[3-(3-Amino-phenyl)-imidazo[1,2-b]pyridazin-6-yl]-(3-chloro-benzyl)-amine;
1-[3-(6-Propylamino-imidazo[1,2-b]pyridazin-3-yl)-phenyl]-ethanone;
[3-(4-Amino-phenyl)-imidazo[1,2-b]pyridazin-6-yl]-propyl-amine;
[3-(4-Chloro-phenyl)-imidazo[1,2-b]pyridazin-6-yl]-(3-morpholin-4-yl-propyl)-amine;
1-{3-[6-(3-Morpholin-4-yl-propylamino)-imidazo[1,2-b]pyridazin-3-yl]-phenyl}-ethanone;
N'-[3-(4-Fluoro-phenyl)-imidazo[1,2-b]pyridazin-6-yl]-N,N-dimethyl-ethane-1,2-diamine;
N,N-Dimethyl-N'-[3-(4-phenoxy-phenyl)-imidazo[1,2-b]pyridazin-6-yl]-ethane-1,2-diamine;
4-[6-(3-Chloro-benzylamino)-imidazo[1,2-b]pyridazin-3-yl]-N-(2-dimethylamino-ethyl)-benzamide;
N-(2-Dimethylamino-ethyl)-3-[6-(4-fluoro-benzylamino)-imidazo [1,2-b]pyridazin-3-yl]-benzamide;
[3-(3-Fluoro-phenyl)-imidazo[1,2-b]pyridazin-6-yl]-(3-morpholin-4-yl-propyl)-amine;
N'-[3-(2-Fluoro-phenyl)-imidazo[1,2-b]pyridazin-6-yl]-N,N-dimethyl-ethane-1,2-diamine;
N,N-Dimethyl-N'-(3-p-tolyl-imidazo[1,2-b]pyridazin-6-yl)-ethane-1,2-diamine;
4-(6-Propylamino-imidazo[1,2-b]pyridazin-3-yl)-benzoic acid;
N'-[3-(3-Chloro-phenyl)-imidazo [1,2-b]pyridazin-6-yl]-N,N-dimethyl-ethane-1,2-diamine;
1-{3-[6-(4-Fluoro-benzylamino)-imidazo[1,2-b]pyridazin-3-yl]-phenyl}-ethanone; and
1-{3-[6-(2-Pyridin-2-yl-ethylamino)-imidazo[1,2-b]pyridazin-3-yl]-phenyl}-ethanone.

Prodrugs of the compounds of the present invention are also within the scope of the present invention.

"Prodrug" means a derivative that is converted into a compound according to the present invention by a reaction with an enzyme, gastric acid or the like under a physiological condition in the living body, e.g. by oxidation, reduction, hydrolysis or the like, each of which is carried out enzymatically. Examples of a prodrug are compounds, wherein the amino group in a compound of the present invention is acylated, alkylated or phosphorylated to form, e.g., eicosanoylamino, alanylamino, pivaloyloxymethylamino or wherein the hydroxyl group is acylated, alkylated, phosphorylated or converted into the borate, e.g. acetyloxy, palmitoyloxy, pivaloyloxy, succinyloxy, fumaryloxy, alanyloxy or wherein the carboxyl group is esterified or amidated. These compounds can be produced from compounds of the present invention according to well-known methods.

Metabolites of compounds of formula (I) are also within the scope of the present invention.

The term "metabolites" refers to all molecules derived from any of the compounds according to the present invention in a cell or organism, preferably mammal.

Preferably the term relates to molecules which differ from any molecule which is present in any such cell or organism under physiological conditions

The structure of the metabolites of the compounds according to the present invention will be obvious to any person skilled in the art, using the various appropriate methods.

Where tautomerism, like e.g. keto-enol tautomerism, of compounds of general formula (I) may occur, the individual forms, like e.g. the keto and enol form, are comprised separately and together as mixtures in any ratio. Same applies for stereoisomers, like e.g. enantiomers, cis/trans isomers, conformers and the like.

If desired, isomers can be separated by methods well known in the art, e.g. by liquid chromatography. Same applies for enantiomers by using e.g. chiral stationary phases. Additionally, enantiomers may be isolated by converting them into diastereomers, i.e. coupling with an enantiomerically pure auxiliary compound, subsequent separation of the resulting diastereomers and cleavage of the auxiliary residue. Alternatively, any enantiomer of a compound of formula (I) may be obtained from stereoselective synthesis using optically pure starting materials.

In case the compounds according to formula (I) contain one or more acidic or basic groups, the invention also comprises their corresponding pharmaceutically or toxicologically acceptable salts, in particular their pharmaceutically utilizable salts. Thus, the compounds of the formula (I) which contain acidic groups can be used according to the invention, for example, as alkali metal salts, alkaline earth metal salts or as ammonium salts. More precise examples of such salts include sodium salts, potassium salts, calcium salts, magnesium salts or salts with ammonia or organic amines such as, for example, ethylamine, ethanolamine, triethanolamine or amino acids. Compounds of the formula (I) which contain one or more basic groups, i.e. groups which can be protonated, can be present and can be used according to the invention in the form of their addition salts with inorganic or organic acids. Examples for suitable acids include hydrogen chloride, hydrogen bromide, phosphoric acid, sulfuric acid, nitric acid, methanesulfonic acid, p-toluenesulfonic acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, and other acids known to the person skilled in the art. If the compounds of the formula (I) simultaneously contain acidic and basic groups in the molecule, the invention also includes, in addition to the salt forms mentioned, inner salts or betaines (zwitterions). The respective salts according to the formula (I) can be obtained by customary methods which are known to the person skilled in the art like, for example by contacting these with an organic or inorganic acid or base in a solvent or dispersant, or by anion exchange or cation exchange with other salts. The present invention also includes all salts of the compounds of the formula (I) which, owing to low physiological compatibility, are not directly suitable for use in pharmaceuticals but which can be used, for example, as intermediates for chemical reactions or for the preparation of pharmaceutically acceptable salts.

The term "pharmaceutically acceptable" means approved by a regulatory agency such as the EMEA (Europe) and/or the FDA (US) and/or any other national regulatory agency for use in animals, preferably in humans.

The present invention furthermore includes all solvates of the compounds according to the invention.

The present invention provides compounds of formula (I) as kinase inhibitors, especially as Itk inhibitors.

Accordingly, the compounds of the present invention are useful for the prevention or treatment of immunological disorders (e.g. immune or autoimmune diseases), inflammatory disorders or allergic disorders.

Thus, another object of the present invention is a compound of the present invention or a pharmaceutically acceptable salt thereof for use as a medicament.

Yet another object of the present invention is the use of a compound of the present invention or a pharmaceutically acceptable salt thereof for the manufacture of a medicament for the treatment or prophylaxis of diseases and disorders associated with Itk kinase.

"Itk" or "Itk kinase" means Interleukin-2 (IL-2)-inducible T-cell kinase (also known as Emt or Tsk).

Yet another object of the present invention is the use of a compound of the present invention or a pharmaceutically acceptable salt thereof for the manufacture of a medicament for the treatment or prophylaxis of immunological, inflammatory or allergic disorders.

Yet another object of the present invention is a method for treating, controlling, delaying or preventing in a mammalian patient in need of treatment one or more conditions selected from the group consisting of immunological, inflammatory, allergic disorders and other diseases and disorders associated with Itk kinase, wherein the method comprises the administration to said patient an therapeutically effective amount of a compound according to the present invention or a pharmaceutically acceptable salt thereof.

As used herein, the term "treating" or "treatment" is intended to refer to all processes, wherein there may be a slowing, interrupting, arresting, or stopping of the progression of a disease, but does not necessarily indicate a total elimination of all symptoms.

Without intending to be limited by theory, the compounds of the invention modulate T cell and mast cell activation via inhibition of Itk. The inhibition of T cell activation is therapeutically useful for suppressing immune functions. Therefore the inhibition of Itk is useful for preventing and treating a variety of immune disorders, including autoimmune diseases, organ and bone marrow transplant rejection, graft-versus-host disease, and inflammatory diseases.

In particular, the compounds of the invention may be used to prevent or treat acute or chronic inflammation, allergies, contact dermatitis, psoriasis, rheumatoid arthritis, multiple sclerosis, type I diabetes, inflammatory bowel disease, Crohn's disease, ulcerative colitis, graft versus host disease and lupus erythematosus.

Inhibitors of mast cell activation and degranulation prevent the release of proinflammatory mediators and cytokines. Thus inhibition of Itk may be used to prevent and treat inflammatory and allergic disorders, including asthma, chronic obstructive pulmonary disease (COPD), acute respiratory distress syndrome (ARDS), bronchitis, conjunctivitis, dermatitis and allergic rhinitis. Other disorders mediated by T cells or mast cells will be known to those of ordinary skill in the art and can also be treated with the compounds of the invention.

Accordingly, an object of the present invention is the use of a compound of the present invention or a pharmaceutically acceptable salt thereof for the manufacture of a medicament for the treatment or prophylaxis of autoimmune diseases, organ and bone marrow transplant rejection, graft-versus-host disease, acute or chronic inflammation, contact dermatitis, psoriasis, rheumatoid arthritis, multiple sclerosis, type I diabetes, inflammatory bowel disease, Crohn's disease, ulcerative colitis, graft versus host disease, lupus erythematosus, asthma, chronic obstructive pulmonary disease (COPD), acute respiratory distress syndrome (ARDS), bronchitis, conjunctivitis, dermatitis or allergic rhinitis.

Yet another object of the present invention is a method for treating, controlling, delaying or preventing in a mammalian patient in need of treatment one or more conditions selected from the group autoimmune diseases, organ and bone marrow transplant rejection, graft-versus-host disease, acute or chronic inflammation, contact dermatitis, psoriasis, rheumatoid arthritis, multiple sclerosis, type I diabetes, inflammatory bowel disease, Crohn's disease, ulcerative colitis, graft versus host disease, lupus erythematosus, asthma, chronic obstructive pulmonary disease (COPD), acute respiratory distress syndrome (ARDS), bronchitis, conjunctivitis, dermatitis and allergic rhinitis, wherein the method comprises the administration to said patient an therapeutically effective amount of a compound according to the present invention or a pharmaceutically acceptable salt thereof.

The present invention provides pharmaceutical compositions comprising a compound of formula (I) or a pharmaceutically acceptable salt thereof as active ingredient together with a pharmaceutically acceptable carrier, optionally in combination with one or more other pharmaceutical compositions.

"Pharmaceutical composition" means one or more active ingredients, and one or more inert ingredients that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing a compound of the present invention and a pharmaceutically acceptable carrier.

The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, including but not limited to peanut oil, soybean oil, mineral oil, sesame oil and the like. Water is a preferred carrier when the pharmaceutical composition is administered orally. Saline and aqueous dextrose are preferred carriers when the pharmaceutical composition is administered intravenously. Saline solutions and aqueous dextrose and glycerol solutions are preferably employed as liquid carriers for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsions, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin. Such compositions will contain a therapeutically effective amount of the therapeutic, preferably in purified form, together with a suitable amount of carrier so as to provide the form for proper administration to the patient. The formulation should suit the mode of administration.

A pharmaceutical composition of the present invention may comprise one or more additional compounds as active ingredients like one or more compounds of formula (I) not being the first compound in the composition or other Itk inhibitors.

Other active ingredients for use in combination with other therapies for the treatment of immune, inflammatory, allergic disorders and may include steroids, leukotriene antagonsits, anti-histamines, cyclosporine or rapamycin.

The pharmaceutical compositions of the present invention include compositions suitable for oral, rectal, topical, parenteral (including subcutaneous, intramuscular, and intravenous), ocular (ophthalmic), pulmonary (nasal or buccal inhalation), or nasal administration, although the most suitable route in any given case will depend on the nature and severity of the conditions being treated and on the nature of the active ingredient. They may be conveniently presented in unit dosage form and prepared by any of the methods well-known in the art of pharmacy.

In practical use, the compounds of formula (I) can be combined as the active ingredient in intimate admixture with a pharmaceutical carrier according to conventional pharmaceutical compounding techniques. The carrier may take a wide variety of forms depending on the form of preparation desired for administration, *e.g.,* oral or parenteral (including intravenous). In preparing the compositions for oral dosage form, any of the usual pharmaceutical media may be employed, such as water, glycols, oils, alcohols, flavoring agents, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as powders, hard and soft capsules and tablets, with the solid oral preparations being preferred over the liquid preparations.

Because of their ease of administration, tablets and capsules represent the most advantageous oral dosage unit form in which case solid pharmaceutical carriers are obviously employed. If desired, tablets may be coated by standard aqueous or non-aqueous techniques. Such compositions and preparations should contain at least 0.1 percent of active compound. The percentage of active compound in these compositions may, of course, be varied and may conveniently be between about 2 percent to about 60 percent of the weight of the unit. The amount of active compound in such therapeutically useful compositions is such that an effective dosage will be obtained. The active compounds can also be administered intranasally, for example, as liquid drops or spray.

The tablets, pills, capsules, and the like may also contain a binder such as gum tragacanth, acacia, corn starch or gelatin; excipients such as dicalcium phosphate; a disintegrating agent such as corn starch, potato starch, alginic acid; a lubricant such as magnesium stearate; and a sweetening agent such as sucrose, lactose or saccharin. When a dosage unit form is a capsule, it may contain, in addition to materials of the above type, a liquid carrier such as a fatty oil.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor.

Compounds of formula (I) may also be administered parenterally. Solutions or suspensions of these active compounds can be prepared in water suitably mixed with a surfactant such as hydroxypropyl-cellulose. Dispersions can also be prepared in glycerol, liquid polyethylene glycols and mixtures thereof in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (*e.g*., glycerol, propylene glycol and liquid polyethylene glycol), suitable mixtures thereof, and vegetable oils.

Any suitable route of administration may be employed for providing a mammal, especially a human, with an effective dose of a compound of the present invention. For example, oral, rectal, topical, parenteral, ocular, pulmonary, nasal, and the like may be employed. Dosage forms include tablets, troches, dispersions, suspensions, solutions, capsules, creams, ointments, aerosols, and the like. Preferably compounds of formula (I) are administered orally.

The effective dosage of active ingredient employed may vary depending on the particular compound employed, the mode of administration, the condition being treated and the severity of the condition being treated. Such dosage may be ascertained readily by a person skilled in the art.

Methods for the synthesis of the compounds of the present invention are described e.g., in Houben-Weyl, Methoden der Organischen Chemie (Methods of Organic Chemistry), Thieme-Verlag, Stuttgart, or Organic Reactions, John Wiley & Sons, New York.

Depending on the circumstances of the individual case, in order to avoid side reactions during the synthesis of a compound of formula (I), it can be necessary or advantageous to temporarily block functional groups by introducing protective groups and to deprotect them in a later stage of the synthesis, or introduce functional groups in the form of precursor groups which in a later stage are converted into the desired functional groups. Such synthesis strategies and protective groups and precursor groups which are suitable in an individual case are known to the person skilled in the art.

If desired, the compounds of the formula (I) can be purified by customary purification procedures, for example by distillation, re-crystallization or chromatography. The starting compounds for the preparation of the compounds of the formula (I) are commercially available or can be prepared according to or analogously to literature procedures.

A general route for the synthesis of compounds or formula (I) may start with compounds of formula (II) which are readily available by conventional methods for the preparation of this type of heterocycle. Such methods are well known for the person skilled in the art.

According to scheme 1 compounds of formula (II), wherein A¹, A², R³ to R⁵ have the meaning as indicated above may react in a first step with compounds of the formula R²-XH, wherein R², X have the meaning as indicated above and the resulting intermediate may react with boronic acid R¹-B(OH)₂, wherein R¹ has the meaning as indicated above, in a Suzuki reaction to yield compounds of formula (I).

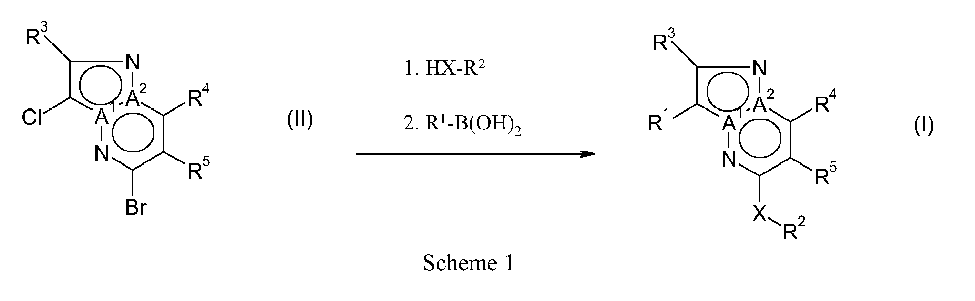

R²-XH and R¹-B(OH)₂ as suitable starting materials for the synthesis of preferred compounds of the present invention may be purchased from commercially available sources such as Array, Sigma Aldrich, Fluka, ABCR or be synthesized by one skilled in the art.

**General procedure A** for the preparation of preferred compounds of formula (Ib):

In a general procedure A for the preparation of preferred compounds of formula (Ib), wherein X is NH and R³ to R⁵ is H, 3-bromo-6-chloroimadazo[1,2-b]pyridazine **(3),** is readily synthesized in two steps. Condensation of commercially available 3-amino-6-chloropyridazine **(1)** and bromoacetaldehyde in propan-2-ol / 48% HBr gives the 6-chloroimadazo[1,2-b]pyridazine **(2).** Bromination at the 6-position by treatment of **(2)** with *N-*bromosuccinimide in chloroform affords 3-bromo-6-chloroimadazo[1,2-b]pyridazine.

Amine displacement using microwave irradiation, followed by Suzuki cross-coupling at the 3-position using the conditions shown in scheme 2, provides the preferred compounds of formula (Ib), wherein R¹ and R² have the meaning as indicated above:

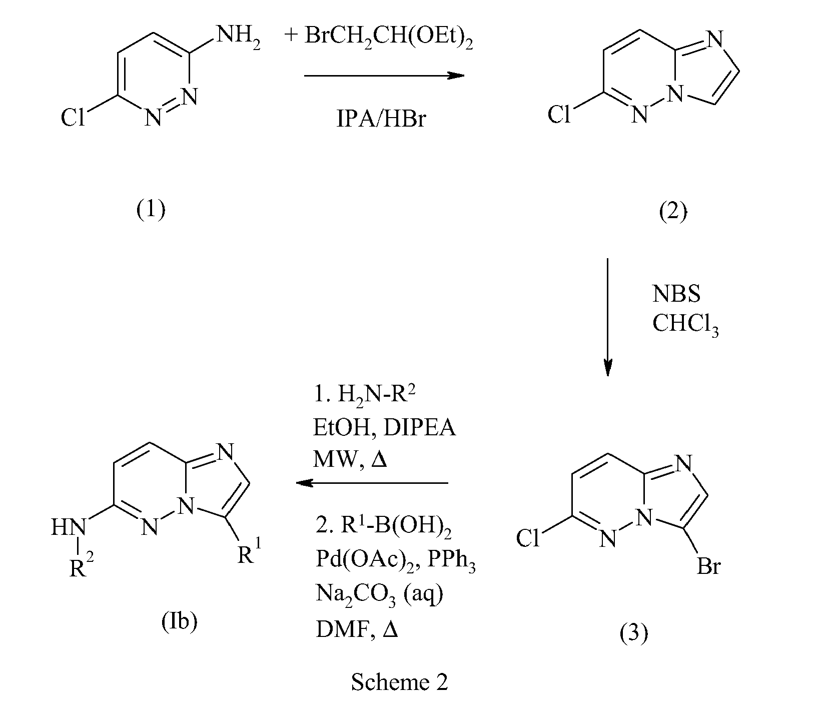

### General procedure B for the preparation of preferred compounds of formula (Ia):

In a general procedure B for the preparation of preferred compounds of formula (Ia), wherein X is NH and R³ to R⁵ is H, reaction of commercially available N,N' dimethyl uracil **(4)** with 3-aminopyrazine **(5)** gives intermediate **(6)** which is converted to the chloro compound **(7)** using phosphorus oxychloride and subsequently brominated using *N*-bromosuccinimide in either *iso*-propylacetate or dichloromethane to give **(8).**

Amine displacement under thermal conditions provids intermediate **(9)** as the sole regioisomer. Where a primary amine is used for initial displacement reaction, it is necessary to Boc protect the amine NH prior to Suzuki coupling. Suzuki coupling of the 3-bromo moiety followed by deprotection of the amine using TFA, where necessary, completes the synthesis as shown in scheme 3 of preferred compounds of formula (Ia), wherein R¹ and R² have the meaning as indicated above:

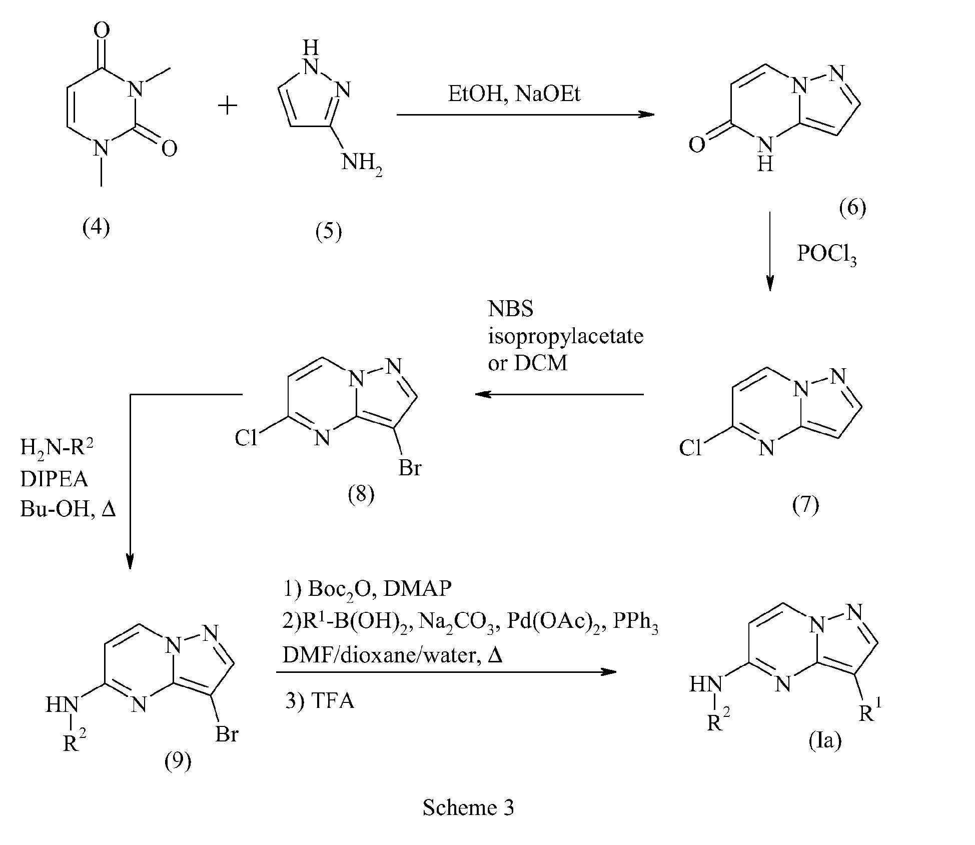

### Abbreviations:

- Ac: acetyl
- Boc: benzyloxy-carbonyl
- DCM: dichloromethane
- DIPEA: diisopropylethylamine
- DMAP: 4-(dimethylamino)-pyridine
- DMF: dimethylformamide
- Et: Ethyl
- IPA: isopropanol
- MW: micro wave
- NBS: N-bromosuccinimide
- Ph: phenyl
- TFA: trifluoroacetic acid

### Examples

### Example 1: Preparation of preferred compounds of the present invention

According to the general procedures A and B described above the following preferred compounds of formulae (Ia) and (Ib) are prepared:
1-{5-[6-(3-Hydroxy-propylamino)-imidazo[1,2-b]pyridazin-3-yl]-thiophen-2-yl}-ethanone;
4-[6-(2-Methoxy-ethylamino)-imidazo[1,2-b]pyridazin-3-yl]-phenol;
4-[6-(Tetrahydro-pyran-4-ylamino)-imidazo[1,2-b]pyridazin-3-yl]-phenol;
(2-Methoxy-ethyl)-[3-(5-methoxy-pyridin-3-yl)-imidazo[1,2-b]pyridazin-6-yl]-amine;
4-[6-(3-Hydroxy-propylamino)-imidazo[1,2-b]pyridazin-3-yl]-benzonitrile;
{4-[6-(Tetrahydro-pyran-4-ylamino)-imidazo[1,2-b]pyridazin-3-yl]-phenyl}-methanol;
{3-[6-(Tetrahydro-pyran-4-ylamino)-imidazo[1,2-b]pyridazin-3-yl]-phenyl}-methanol;
3-(3-Furan-3-yl-imidazo[1,2-b]pyridazin-6-ylamino)-propan-1-ol;
2-Methoxy-4-[6-(tetrahydro-pyran-4-ylamino)-imidazo[1,2-b]pyridazin-3-yl]-phenol;
3-[6-(1-Hydroxymethyl-propylamino)-imidazo[1,2-b]pyridazin-3-yl]-benzamide;
N-{3-[6-(1-Hydroxymethyl-propylamino)-imidazo[1,2-b]pyridazin-3-yl]-phenyl}-methanesulfonamide;
[3-(4-Morpholin-4-yl-phenyl)-imidazo[1,2-b]pyridazin-6-yl]-(2-pyridin-2-yl-ethyl)-amine;
(3-Pyridin-4-yl-imidazo[1,2-b]pyridazin-6-yl)-thiophen-2-ylmethyl-amine;
1-(5-{6-[(Pyridin-2-ylmethyl)-amino]-imidazo[1,2-b]pyridazin-3-yl}-thiophen-2-yl)-ethanone;
Furan-2-ylmethyl-(3-pyridin-3-yl-imidazo[1,2-b]pyridazin-6-yl)-amine;
(3-Pyridin-3-yl-imidazo[1,2-b]pyridazin-6-yl)-thiophen-2-ylmethyl-amine;
Furan-2-ylmethyl-(3-pyridin-4-yl-imidazo[1,2-b]pyridazin-6-yl)-amine;
1-(5-{6-[(Thiophen-2-ylmethyl)-amino]-imidazo[1,2-b]pyridazin-3-yl}-thiophen-2-yl)-ethanone;
4-[3-(3-Trifluoromethoxy-phenyl)-imidazo[1,2-b]pyridazin-6-ylamino]-cyclohexanol;
4-[3-(1H-Indol-5-yl)-imidazo[1,2-b]pyridazin-6-ylamino]-cyclohexanol;
4-[3-(3,4-Dimethoxy-phenyl)-imidazo [1,2-b]pyridazin-6-ylamino]-cyclohexanol;
N-{3-[6-(4-Hydroxy-cyclohexylamino)-imidazo[1,2-b]pyridazin-3-yl]-phenyl}-acetamide;
(3-Pyrimidin-5-yl-imidazo[1,2-b]pyridazin-6-yl)-thiophen-2-ylmethyl-amine;
3-[6-((S)-1-Hydroxymethyl-2-methyl-propylamino)-imidazo[1,2-b]pyridazin-3-yl]-benzamide;
[3-(4-Methyl-thiophen-2-yl)-imidazo[1,2-b]pyridazin-6-yl]-pyridin-2-ylmethyl-amine;
(S)-3-Methyl-2-(3-pyrimidin-5-yl-imidazo[1,2-b]pyridazin-6-ylamino)-butan-1-ol;
4-[6-((S)-1-Hydroxymethyl-2-methyl-propylamino)-imidazo[1,2-b]pyridazin-3-yl]-2-methoxy-phenol;
4-[3-(3-Dimethylamino-phenyl)-imidazo[1,2-b]pyridazin-6-ylamino]-cyclohexanol;
Furan-2-ylmethyl-(3-pyrimidin-5-yl-imidazo[1,2-b]pyridazin-6-yl)-amine;
4-[6-(4-Hydroxy-cyclohexylamino)-imidazo[1,2-b]pyridazin-3-yl]-benzonitrile;
3-[6-(4-Hydroxy-cyclohexylamino)-imidazo[1,2-b]pyridazin-3-yl]-benzoic acid;
4-[3-(4-Methoxy-phenyl)-imidazo[1,2-b]pyridazin-6-ylamino]-cyclohexanol;
4-(3-Thiophen-3-yl-imidazo[1,2-b]pyridazin-6-ylamino)-cyclohexanol;
4-[6-(4-Hydroxy-cyclohexylamino)-imidazo[1,2-b]pyridazin-3-yl]-benzoic acid;
4-[3-(4-Hydroxymethyl-phenyl)-imidazo[1,2-b]pyridazin-6-ylamino]-cyclohexanol;
4-[6-(4-Hydroxy-cyclohexylamino)-imidazo[1,2-b]pyridazin-3-yl]-2-methoxy-phenol;
1-{5-[6-(4-Hydroxy-cyclohexylamino)-imidazo[1,2-b]pyridazin-3-yl]-thiophen-2-yl}-ethanone;
4-(6-Propylamino-imidazo[1,2-b]pyridazin-3-yl)-phenol;
N,N-Dimethyl-N'-(3-thiophen-3-yl-imidazo[1,2-b]pyridazin-6-yl)-ethane-1,2-diamine;
4-[3-(2,4-Dimethoxy-pyrimidin-5-yl)-imidazo[1,2-b]pyridazin-6-ylamino]-cyclohexanol;
N-[3-(6-Propylamino-imidazo[1,2-b]pyridazin-3-yl)-phenyl]-acetamide;
N'-(3-Benzo[b]thiophen-2-yl-imidazo[1,2-b]pyridazin-6-yl)-N,N-dimethyl-ethane-1,2-diamine;
(2,4-Dimethyl-benzyl)-(3-thiophen-3-yl-imidazo[1,2-b]pyridazin-6-yl)-amine;
1-{5-[6-(3-Morpholin-4-yl-propylamino)-imidazo[1,2-b]pyridazin-3-yl]-thiophen-2-yl}-ethanone;
N'-[3-(3,4-Dimethoxy-phenyl)-imidazo[1,2-b]pyridazin-6-yl]-N,N-dimethyl-ethane-1,2-diamine;
(3-Chloro-benzyl)-(3-pyridin-4-yl-imidazo[1,2-b]pyridazin-6-yl)-amine;
(3-Benzo[1,3]dioxol-5-yl-imidazo[1,2-b]pyridazin-6-yl)-(3-morpholin-4-yl-propyl)-amine;
4-[3-(3-Hydroxymethyl-phenyl)-imidazo[1,2-b]pyridazin-6-ylamino]-cyclohexanol;
4-(3-Phenyl-imidazo[1,2-b]pyridazin-6-ylamino)-cyclohexanol;
(3-Morpholin-4-yl-propyl)-(3-thiophen-3-yl-imidazo[1,2-b]pyridazin-6-yl)-amine;
(2,4-Dimethyl-benzyl)-(3-pyridin-4-yl-imidazo[1,2-b]pyridazin-6-yl)-amine;
4-[6-(3-Morpholin-4-yl-propylamino)-imidazo[1,2-b]pyridazin-3-yl]-phenol;
4-[3-(3-Fluoro-phenyl)-imidazo[1,2-b]pyridazin-6-ylamino]-cyclohexanol;
N'-(3-Benzofuran-2-yl-imidazo[1,2-b]pyridazin-6-yl)-N,N-dimethyl-ethane-1,2-diamine;
3-(6-Propylamino-imidazo[1,2-b]pyridazin-3-yl)-benzamide;
[4-(6-Propylamino-imidazo[1,2-b]pyridazin-3-yl)-phenyl]-methanol;
Propyl-(3-pyrimidin-5-yl-imidazo[1,2-b]pyridazin-6-yl)-amine;
3-[6-(3-Morphohn-4-yl-propylamino)-imidazo [1,2-b]pyridazin-3-yl]-phenol;
2-Methoxy-4-(6-propylamino-imidazo[1,2-b]pyridazin-3-yl)-phenol;
1-{5-[5-(2-Hydroxy-ethylamino)-pyrazolo[1,5-a]pyrimidin-3-yl]-thiophen-2-yl}-ethanone;
N-{3-[5-(2-Hydroxy-ethylamino)-pyrazolo[1,5-a]pyrimidin-3-yl]-phenyl}-acetamide;
3-[6-(2-Dimethylamino-ethylamino)-imidazo [1,2-b]pyridazin-3-yl]-benzamide;
4-[3-(3-Methoxy-phenyl)-imidazo[1,2-b]pyridazin-6-ylamino]-cyclohexanol;
2-(3-Benzo[1,3]dioxol-5-yl-pyrazolo[1,5-a]pyrimidin-5-ylamino)-ethanol;
4-{5-[(Pyridin-4-ylmethyl)-amino]-pyrazolo[1,5-a]pyrimidin-3-yl}-phenol;
4-(3-Pyridin-3-yl-pyrazolo[1,5-a]pyrimidin-5-ylamino)-cyclohexanol;
3-[5-(4-Acetylamino-phenylamino)-pyrazolo[1,5-a]pyrimidin-3-yl]-benzoic acid;
4-[3-(3,4-Dimethoxy-phenyl)-pyrazolo[1,5-a]pyrimidin-5-ylamino]-cyclohexanol;
2-[3-(3,4-Dimethoxy-phenyl)-pyrazolo[1,5-a]pyrimidin-5-ylamino]-ethanol;
4-[5-(4-Hydroxy-cyclohexylamino)-pyrazolo[1,5-a]pyrimidin-3-yl]-phenol;
(1H-Indol-5-yl)-[3-(5-methoxy-pyridin-3-yl)-pyrazolo[1,5-a]pyrimidin-5-yl]-amine;
4-[5-(2-Hydroxy-ethylamino)-pyrazolo [1,5-a]pyrimidin-3-yl]-benzonitrile;
2-[3-(3,4,5-Trimethoxy-phenyl)-pyrazolo [1,5-a]pyrimidin-5-ylamino]-ethanol;
2-Methoxy-4-[5-(3,4,5-trimethoxy-phenylamino)-pyrazolo [1,5-a]pyrimidin-3-yl]-phenol;
4-[3-(6-Methoxy-pyridin-3-yl)-pyrazolo[1,5-a]pyrimidin-5-ylamino]-phenol;
4-[5-(2-Dimethylamino-ethylamino)-pyrazolo[1,5-a]pyrimidin-3-yl]-benzoic acid;
[3-(4-Chloro-phenyl)-pyrazolo[1,5-a]pyrimidin-5-yl]-pyridin-3-ylmethyl-amine;
3-[5-(6-Methoxy-pyridin-3-ylamino)-pyrazolo[1,5-a]pyrimidin-3-yl]-phenol;
(6-Methoxy-pyridin-3-yl)-[3-(6-methoxy-pyridin-3-yl)-pyrazolo[1,5-a]pyrimidin-5-yl]-amine;
4-[3-(6-Methoxy-pyridin-3-yl)-pyrazolo[1,5-a]pyrimidin-5-ylamino]-cyclohexanol;
4-[5-(4-Hydroxy-cyclohexylamino)-pyrazolo[1,5-a]pyrimidin-3-yl]-2-methoxy-phenol;
3-{5-[(Pyridin-3-ylmethyl)-amino]-pyrazolo[1,5-a]pyrimidin-3-yl}-phenol;
(3-{5-[(Pyridin-3-ylmethyl)-amino]-pyrazolo[1,5-a]pyrimidin-3-yl}-phenyl)-methanol;
3-[5-(4-Hydroxy-cyclohexylamino)-pyrazolo[1,5-a]pyrimidin-3-yl]-phenol;
2-[3-(3-Hydroxymethyl-phenyl)-pyrazolo[1,5-a]pyrimidin-5-ylamino]-ethanol;
N'-[3-(4-Methoxy-phenyl)-pyrazolo[1,5-a]pyrimidin-5-yl]-N,N-dimethyl-ethane-1,2-diamine;
N- {5-[3-(4-Hydroxy-3-methoxy-phenyl)-pyrazolo[1,5-a]pyrimidin-5-ylamino]-2-methylphenyl}-methanesulfonamide;
(E)-3-{3-[5-(4-Hydroxy-cyclohexylamino)-pyrazolo[1,5-a]pyrimidin-3-yl]-phenyl}-acrylic acid;
2-[3-(6-Methoxy-pyridin-3-yl)-pyrazolo[1,5-a]pyrimidin-5-ylamino]-ethanol;
3-[5-(2-Dimethylamino-ethylamino)-pyrazolo[1,5-a]pyrimidin-3-yl]-phenol;
[3-(1-Methyl-1H-pyrazol-4-yl)-pyrazolo[1,5-a]pyrimidin-5-yl]-(3,4,5-trimethoxy-phenyl)-amine;
4-[3-(3-Hydroxymethyl-phenyl)-pyrazolo[1,5-a]pyrimidin-5-ylamino]-cyclohexanol;
4-[5-(2-Hydroxy-ethylamino)-pyrazolo[1,5-a]pyrimidin-3-yl]-2-methoxy-phenol;
3-[5-(3,4,5-Trimethoxy-phenylamino)-pyrazolo[1,5-a]pyrimidin-3-yl]-benzamide;
4-[3-(2-Hydroxymethyl-phenyl)-pyrazolo[1,5-a]pyrimidin-5-ylamino]-cyclohexanol;
3-[5-(4-Hydroxy-cyclohexylamino)-pyrazolo[1,5-a]pyrimidin-3-yl]-benzamide;
1-{3-[5-(4-Morpholin-4-yl-phenylamino)-pyrazolo[1,5-a]pyrimidin-3-yl]-phenyl}-ethanone;
Phenyl-(3-pyridin-4-yl-pyrazolo[1,5-a]pyrimidin-5-yl)-amine;
[3-(1-Methyl-1H-pyrazol-4-yl)-pyrazolo[1,5-a]pyrimidin-5-yl]-(4-morpholin-4-yl-phenyl)-amine;
3-{5-[(Pyridin-3-ylmethyl)-amino]-pyrazolo[1,5-a]pyrimidin-3-yl}-benzamide;
1-(5-{5-[(Pyridin-3-ylmethyl)-amino]-pyrazolo[1,5-a]pyrimidin-3-yl}-thiophen-2-yl)-ethanone;
N'-(3-Benzo[1,3]dioxol-5-yl-pyrazolo[1,5-a]pyrimidin-5-yl)-N,N-dimethyl-ethane-1,2-diamine;
N-(2-Dimethylamino-ethyl)-3-{5-[(pyridin-3-ylmethyl)-amino]-pyrazolo[1,5-a]pyrimidin-3-yl}-benzamide;
N-(2-Dimethylamino-ethyl)-4-{5-[(pyridin-3-ylmethyl)-amino]-pyrazolo[1,5-a]pyrimidin-3-yl}-benzamide;
4-{5-[(Pyridin-3-ylmethyl)-amino]-pyrazolo[1,5-a]pyrimidin-3-yl}-phenol;
N-(2-Dimethylamino-ethyl)-3-[5-(6-methoxy-pyridin-3-ylamino)-pyrazolo[1,5-a]pyrimidin-3-yl]-benzamide;
1-{5-[5-(2-Dimethylamino-ethylamino)-pyrazolo[1,5-a]pyrimidin-3-yl]-thiophen-2-yl}-ethanone;
4-[3-(3-Aminomethyl-phenyl)-pyrazolo[1,5-a]pyrimidin-5-ylamino]-phenol;
4-[3-(4-Fluoro-phenyl)-pyrazolo[1,5-a]pyrimidin-5-ylamino]-cyclohexanol;
[3-(3-Chloro-phenyl)-pyrazolo[1,5-a]pyrimidin-5-yl]-(4-morpholin-4-yl-phenyl)-amine;
[3-(3-Chloro-phenyl)-pyrazolo[1,5-a]pyrimidin-5-yl]-pyridin-3-ylmethyl-amine;
[3-(3-Amino-phenyl)-imidazo[1,2-b]pyridazin-6-yl]-(3-chloro-benzyl)-amine;
1-[3-(6-Propylamino-imidazo[1,2-b]pyridazin-3-yl)-phenyl]-ethanone;
[3-(4-Amino-phenyl)-imidazo[1,2-b]pyridazin-6-yl]-propyl-amine;
[3-(4-Chloro-phenyl)-imidazo[1,2-b]pyridazin-6-yl]-(3-morpholin-4-yl-propyl)-amine;
1-{3-[6-(3-Morpholin-4-yl-propylamino)-imidazo[1,2-b]pyridazin-3-yl]-phenyl}-ethanone;
N'-[3-(4-Fluoro-phenyl)-imidazo[1,2-b]pyridazin-6-yl]-N,N-dimethyl-ethane-1,2-diamine;
N,N-Dimethyl-N'-[3-(4-phenoxy-phenyl)-imidazo[1,2-b]pyridazin-6-yl]-ethane-1,2-diamine;
4-[6-(3-Chloro-benzylamino)-imidazo[1,2-b]pyridazin-3-yl]-N-(2-dimethylamino-ethyl)-benzamide;
N-(2-Dimethylamino-ethyl)-3-[6-(4-fluoro-benzylamino)-imidazo[1,2-b]pyridazin-3-yl]-benzamide;
[3-(3-Fluoro-phenyl)-imidazo[1,2-b]pyridazin-6-yl]-(3-morpholin-4-yl-propyl)-amine;
N'-[3-(2-Fluoro-phenyl)-imidazo[1,2-b]pyridazin-6-yl]-N,N-dimethyl-ethane-1,2-diamine;
N,N-Dimethyl-N'-(3-p-tolyl-imidazo[1,2-b]pyridazin-6-yl)-ethane-1,2-diamine;
4-(6-Propylamino-imidazo[1,2-b]pyridazin-3-yl)-benzoic acid;
N'-[3-(3-Chloro-phenyl)-imidazo[1,2-b]pyridazin-6-yl]-N,N-dimethyl-ethane-1,2-diamine;
1-{3-[6-(4-Fluoro-benzylamino)-imidazo[1,2-b]pyridazin-3-yl]-phenyl}-ethanone; and
1-{3-[6-(2-Pyridin-2-yl-ethylamino)-imidazo[1,2-b]pyridazin-3-yl]-phenyl}-ethanone.

### Example 2: Determination of the effect of the compounds according to the invention on Itk

Kinase activity and compound inhibition of Itk (recombinant human Itk, GST-tagged; catalogue number V4193, Invitrogen, Carlsbad, CA, USA) is determined using the Z'-LYTE Kinase assay kit - Tyr 1 peptide (catalogue number PV3190) according to the manufacturer's instructions (Invitrogen, Carlsbad, CA, USA).

The Z'-LYTE biochemical assay uses a fluorescence resonance energy transfer (FRET) - based coupled enzyme format that is based on the differential sensitivity of phosphorylated and non-phosphorylated peptides to proteolytic cleaveage. The peptide substrate is labeled with two fluorophores constituting a FRET pair. In the primary reaction, the kinase transfers the gamma-phosphate of ATP to a tyrosine residue in the synthetic peptide substrate. In the secondary development reaction, a site-specific protease cleaves non-phosphorylated peptides. Cleavage disrupts FRET between the donor and acceptor fluorophores on the peptide, whereas uncleaved phosphorylated peptides maintain FRET.

Calculation of the ratio of donor emission to acceptor emission (after excitation of the donor at 400 nm) quantifies the reaction progress (Rodems et al., 2002, Assay Drug Dev. Technol. 1, 9-19).

Stock solutions of compounds (1.6 mM in DMSO) are diluted in 2% DMSO so that final concentration of 8 µM and 0.8 µM are achieved in the assay.

In general, compounds of the invention are effective for the inhibition of Itk.

## Claims

1. A compound of formula (I) 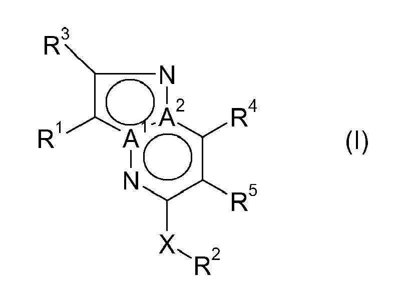 or a pharmaceutically acceptable salt, prodrug or metabolite thereof, wherein
one of A¹, A² is N and the other is C;
X is O; S or NR⁶;
R¹ is T¹;
R² is T²; C₁₋₆ alkyl; C(O)OR⁷; C(O)R⁷; C(O)N(R⁷R^{7a}); S(O)₂N(R⁷R^{7a}); S(O)N(R⁷R^{7a}); S(O)₂R⁷; or S(O)R⁷, wherein C₁₋₆ alkyl is optionally substituted with one or more R⁸;
R³, R⁴, R⁵ are independently selected from the group consisting of H; halogen; CN; C(O)OR⁹; OR⁹; C(O)R⁹; C(O)N(R⁹R^{9a}); S(O)₂N(R⁹R^{9a}); S(O)N(R⁹R^{9a}); S(O)₂R⁹; S(O)R⁹; N(R⁹)S(O)₂N(R^{9a}R^{9b}); SR⁹; N(R⁹R^{9a}); OC(O)R⁹; N(R⁹)C(O)R^{9a}; N(R⁹)S(O)₂R^{9a}; N(R⁹)S(O)R^{9a}; N(R⁹)C(O)N(R^{9a}R^{9b}); N(R⁹)C(O)OR^{9a}; OC(O)N(R⁹R^{9a}); and C₁₋₆ alkyl, wherein C₁₋₆ alkyl is optionally substituted with one or more halogen, which are the same or different;
R⁶, R^{7a}, R⁹, R^{9a}, R^{9b} are independently selected from the group consisting of H; and C₁₋₆ alkyl, wherein C₁₋₆ alkyl is optionally substituted with one or more halogen, which are the same or different;
R⁷ is T²; or C₁₋₆ alkyl, wherein C₁₋₆ alkyl is optionally substituted with one or more R⁸;
T¹ is C₃₋₇ cycloalkyl; heterocyclyl; heterobicyclyl; phenyl; naphthyl; indenyl; indanyl; tetralinyl; decalinyl; or adamantyl, wherein T¹ is optionally substituted with one or more R¹⁰;
T² is C₃₋₇ cycloalkyl; heterocyclyl; heterobicyclyl; phenyl; naphthyl; indenyl; indanyl; tetralinyl; decalinyl; or adamantyl, wherein T² is optionally substituted with one or more R¹¹;
R⁸ is T²; C₁₋₆ alkyl; halogen; CN; C(O)OR¹²; OR¹²; C(O)R¹²; C(O)N(R¹²R^{12a}); S(O)₂N(R¹²R^{12a}); S(O)N(R¹²R^{12a}); S(O)₂R¹²; S(O)R¹²; N(R¹²)S(O)₂N(R^{12a}R^{12b}); SR¹²; N(R¹²R^{12a}); OC(O)R¹²; N(R¹²)C(O)R^{12a}; N(R¹²)S(O)₂R^{12a}; N(R¹²)S(O)R^{12a}; N(R¹²)C(O)N(R^{12a}R^{12b}); N(R¹²)C(O)OR^{12a}; or OC(O)N(R¹²R^{12a}), wherein C₁₋₆ alkyl is optionally substituted with one or more R¹³;
R¹⁰, R¹¹ are independently selected from the group consisting of T³; C₁₋₆ alkyl; halogen; CN; C(O)OR¹²; OR¹²; oxo (=O), where the ring is at least partially saturated; C(O)R¹²; C(O)N(R¹²R^{12a}); S(O)₂N(R¹²R^{12a}); S(O)N(R¹²R^{12a}); S(O)₂R¹²; S(O)R¹²; N(R¹²)S(O)₂N(R^{12a}R^{12b}); SR¹²; N(R¹²R^{12a}); OC(O)R¹²; N(R¹²)C(O)R^{12a}; N(R¹²)S(O)₂R^{12a}; N(R¹²)S(O)R^{12a}; N(R¹²)C(O)N(R^{12a}R^{12b}); N(R¹²)C(O)OR^{12a}; and OC(O)N(R¹²R^{12a}), wherein C₁₋₆ alkyl is optionally substituted with one or more R¹³;
R¹², R^{12a}, R^{12b} are independently selected from the group consisting of H; T³; and C₁₋₆ alkyl, wherein C₁₋₆ alkyl is optionally substituted with one or more R¹⁴;
T³ is C₃₋₇ cycloalkyl; heterocyclyl; heterobicyclyl; phenyl; naphthyl; indenyl; indanyl; tetralinyl; decalinyl; or adamantyl, wherein T³ is optionally substituted with one or more R¹⁵;
R¹³, R¹⁴ are independently selected from the group consisting of halogen; CN; C(O)OR¹⁶; OR¹⁶; C(O)R¹⁶; C(O)N(R¹⁶R^{16a}); S(O)₂N(R¹⁶R^{16a}); S(O)N(R¹⁶R^{16a}); S(O)₂R¹⁶; S(O)R¹⁶; N(R¹⁶)S(O)₂N(R^{16a}R^{16b}); SR¹⁶; N(R¹⁶R^{16a}); OC(O)R¹⁶; N(R¹⁶)C(O)R^{16a}; N(R¹⁶)S(O)₂R^{16a}; N(R¹⁶)S(O)R^{16a}; N(R¹⁶)C(O)N(R^{16a}R^{16b}); N(R¹⁶)C(O)OR^{16a}; OC(O)N(R¹⁶R^{16a}); and C₁₋₆ alkyl, wherein C₁₋₆ alkyl is optionally substituted with one or more halogen, which are the same or different;
R¹⁵ is halogen; CN; C(O)OR¹⁶; OR¹⁶; oxo (=O), where the ring is at least partially saturated; C(O)R¹⁶; C(O)N(R¹⁶R^{16a}); S(O)₂N(R¹⁶R^{16a}); S(O)N(R¹⁶R^{16a}); S(O)₂R¹⁶; S(O)R¹⁶; N(R¹⁶)S(O)₂N(R^{16a} R^{16b}); SR¹⁶; N(R¹⁶R^{16a}); OC(O)R¹⁶; N(R¹⁶)C(O)R^{16a}; N(R¹⁶)S(O)₂R^{16a}; N(R¹⁶)S(O)R^{16a}; N(R¹⁶)C(O)N(R^{16a}R^{16b}); N(R¹⁶)C(O)OR^{16a}; OC(O)N(R¹⁶R^{16a}); or C₁₋₆ alkyl, wherein C₁₋₆ alkyl is optionally substituted with one or more halogen, which are the same or different;
R¹⁶, R^{16a}, R^{16b} are independently selected from the group consisting of H; and C₁₋₆ alkyl, wherein C₁₋₆ alkyl is optionally substituted with one or more halogen, which are the same or different.

2. A compound according to claim 1 of formula (Ia) 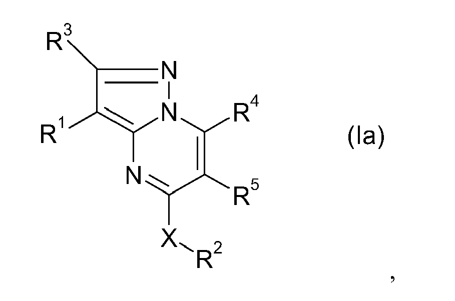 wherein X, R¹ to R⁵ have the meaning as indicated in claim 1.

3. A compound according to claim 1 of formula (Ib) 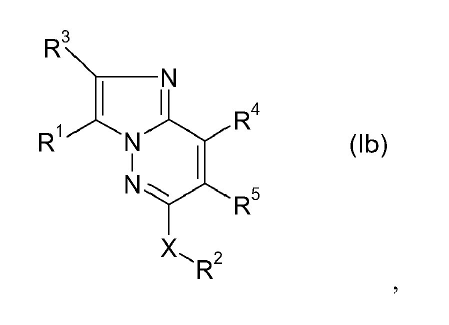 wherein X, R¹ to R⁵ have the meaning as indicated in claim 1.

4. A compound according to any of claims 1 to 3, wherein X is N(R⁶) and wherein R⁶ has the meaning as indicated in claim 1.

5. A compound according to any of claims 1 to 4, wherein R¹ is T¹ and T¹ is heterocyclyl; phenyl; or heterobicyclyl.

6. A compound according to any of claims 1 to 5, wherein R¹ is T¹ and T¹ is unsubstituted aromatic heterocyclyl; substituted aromatic heterocyclyl; substituted phenyl; unsubstituted heterobicyclyl; or substituted heterobicyclyl.

7. A compound according to any of claims 1 to 6, wherein R¹ is T¹ and T¹ is substituted thienyl; unsubstituted thienyl; substituted benzodioxolyl; unsubstituted benzodioxolyl; substituted pyridyl; unsubstituted pyridyl; substituted pyrazolyl; unsubstituted pyrazolyl; substituted indolyl; unsubstituted indolyl; substituted pyrimidyl; unsubstituted pyrimidyl; substituted benzothienyl; unsubstituted benzothienyl; substituted benzofuranyl; unsubstituted benzofuranyl; or substituted phenyl.

8. A compound according to any of claims 1 to 7, wherein T¹ is unsubstituted or substituted with up to three R¹⁰, which are the same or different.

9. A compound according to any of claims 1 to 8, wherein R¹⁰ is C(O)R¹²; C(O)OR¹²; OR¹²; N(R¹²R^{12a}); C(O)N(R¹²R^{12a}); N(R¹²)C(O)R^{12a}; CN; N(R¹²)S(O)₂R^{12a}; T³; C₁₋₆ alkyl; C₁₋₆ alkyl substituted with up to three R¹³, which are the same or different; or halogen.

10. A compound according to claim 9, wherein R¹⁰ is C(O)CH₃; C(O)OH; OH; OCH₃; OCF₃; CH₂OH; C(O)NH₂; C(O)NHCH₂CH₂N(CH₃)₂; NHC(O)CH₃; NHS(O)₂CH₃; CH₃; NH₂; NHCH₃; N(CH₃)₂; (CH₃)₂NCH₂CH₂NH; CH=CHC(O)OH; F; or Cl.

11. A compound according to any of claims 1 to 10, wherein R¹², R^{12a} are independently selected from the group consisting of H; unsubstituted C₁₋₆ alkyl; C₁₋₆ alkyl substituted with up to three R¹³, which are the same or different; and T³.

12. A compound according to any of claims 1 to 11, wherein R¹³ is OH; OCH₃; F; NH₂; NHCH₃; N(CH₃)₂; C(O)OH; or C(O)OCH₃.

13. A compound according to any of claims 1 to 12, wherein R² is T²; C₁₋₆ alkyl; or C₁₋₆ alkyl substituted with up to three R⁸.

14. A compound according to any of claims 1 to 13, wherein R² is C₁₋₆ alkyl substituted with one R⁸.

15. A compound according to any of claims 1 to 14, wherein R² is [C(R¹⁷R^{17a})]ₙCHR⁸ and wherein n is 0, 1 or 2 and wherein R¹⁷, R^{17a} are independently selected from the group consisting of H; CH₃; CH₂CH₃; CH₂CH₂CH₃ and CH₃CHCH₃, provided that the total number of carbon atoms for the C(R¹⁷R^{17a}) group or groups does not exceed 5.

16. A compound according to any of claims 1 to 15, wherein R⁸ is T²; OR¹² ; or N(R¹²R¹²a).

17. A compound according to any of claims 1 to 16, wherein R⁸ is T²; OH; OCH₃; NH₂; NHCH₃; or N(CH₃)₂.

18. A compound according to any of claims 1 to 17, wherein T² is substituted heterocyclyl; unsubstituted heterocyclyl; substituted C₃₋₇ cycloalkyl; unsubstituted phenyl; substituted phenyl; substituted heterobicyclyl; or unsubstituted heterobicyclyl.

19. A compound according to claim 18, wherein T² is substituted pyridyl; unsubstituted pyridyl; substituted cyclohexyl; unsubstituted phenyl; substituted phenyl; substituted indolyl; unsubstituted indolyl; substituted pyranyl; unsubstituted pyranyl; substituted thienyl; unsubstituted thienyl; substituted furyl; unsubstituted furyl; substituted benzothienyl; unsubstituted benzothienyl; substituted morpholinyl; or unsubstituted morpholinyl.

20. A compound according to any of claims 1 to 19, wherein R¹¹ is N(R¹²)C(O)R^{12a}; OR¹²; halogen; C₁₋₆ alkyl; N(R¹²)S(O)₂R^{12a}; or T³.

21. A compound according to any of claims 1 to 20, wherein R¹¹ is NHC(O)CH₃; OH; OCH₃; F; Cl; CH₃; CH₂CH₃; NHS(O)₂CH₃; or T³.

22. A compound according to any of claims 1 to 21, wherein R¹², R^{12a} are independently selected from the group consisting of H; and C₁₋₆ alkyl.

23. A compound according to any of claims 1 to 22, wherein T³ is heterocyclyl; or phenyl.

24. A compound according to any of claims 1 to 23, wherein R³; R⁴; R⁵ are independently selected from the group consisting of H; F; Cl; C(O)OH; C(O)OCH₃; OH; OCH₃; C(O)CH₃; C(O)NH₂; C(O)NHCH₃; C(O)N(CH₃)₂; NH₂; NHCH₃; N(CH₃)₂; and CH₃.

25. A compound according to claim 24, wherein R³; R⁴; R⁵ are H.

26. A compound according to claim 1 selected from the group consisting of
1-{5-[6-(3-Hydroxy-propylamino)-imidazo[1,2-b]pyridazin-3-yl]-thiophen-2-yl}-ethanone;
4-[6-(2-Methoxy-ethylamino)-imidazo[1,2-b]pyridazin-3-yl]-phenol;
4-[6-(Tetrahydro-pyran-4-ylamino)-imidazo[1,2-b]pyridazin-3-yl]-phenol;
(2-Methoxy-ethyl)-[3-(5-methoxy-pyridin-3-yl)-imidazo[1,2-b]pyridazin-6-yl]-amine;
4-[6-(3-Hydroxy-propylamino)-imidazo[1,2-b]pyridazin-3-yl]-benzonitrile;
{4-[6-(Tetrahydro-pyran-4-ylamino)-imidazo[1,2-b]pyridazin-3-yl]-phenyl}-methanol;
{3-[6-(Tetrahydro-pyran-4-ylamino)-imidazo[1,2-b]pyridazin-3-yl]-phenyl}-methanol;
3-(3-Furan-3-yl-imidazo[1,2-b]pyridazin-6-ylamino)-propan-1-ol;
2-Methoxy-4-[6-(tetrahydro-pyran-4-ylamino)-imidazo[1,2-b]pyridazin-3-yl]-phenol;
3-[6-(1-Hydroxymethyl-propylamino)-imidazo[1,2-b]pyridazin-3-yl]-benzamide;
N-{3-[6-(1-Hydroxymethyl-propylamino)-imidazo[1,2-b]pyridazin-3-yl]-phenyl}-methanesulfonamide;
[3-(4-Morpholin-4-yl-phenyl)-imidazo[1,2-b]pyridazin-6-yl]-(2-pyridin-2-yl-ethyl)-amine;
(3-Pyridin-4-yl-imidazo[1,2-b]pyridazin-6-yl)-thiophen-2-ylmethyl-amine;
1-(5-{6-[(Pyridin-2-ylmethyl)-amino]-imidazo[1,2-b]pyridazin-3-yl}-thiophen-2-yl)-ethanone;
Furan-2-ylmethyl-(3-pyridin-3-yl-imidazo[1,2-b]pyridazin-6-yl)-amine;
(3-Pyridin-3-yl-imidazo[1,2-b]pyridazin-6-yl)-thiophen-2-ylmethyl-amine;
Furan-2-ylmethyl-(3-pyridin-4-yl-imidazo[1,2-b]pyridazin-6-yl)-amine;
1-(5-{6-[(Thiophen-2-ylmethyl)-amino]-imidazo[1,2-b]pyridazin-3-yl}-thiophen-2-yl)-ethanone;
4-[3-(3-Trifluoromethoxy-phenyl)-imidazo[1,2-b]pyridazin-6-ylamino]-cyclohexanol;
4-[3-(1H-Indol-5-yl)-imidazo[1,2-b]pyridazin-6-ylamino]-cyclohexanol;
4-[3-(3,4-Dimethoxy-phenyl)-imidazo[1,2-b]pyridazin-6-ylamino]-cyclohexanol;
N-{3-[6-(4-Hydroxy-cyclohexylamino)-imidazo[1,2-b]pyridazin-3-yl]-phenyl}-acetamide;
(3-Pyrimidin-5-yl-imidazo[1,2-b]pyridazin-6-yl)-thiophen-2-ylmethyl-amine;
3-[6-((S)-1-Hydroxymethyl-2-methyl-propylamino)-imidazo[1,2-b]pyridazin-3-yl]-benzamide;
[3-(4-Methyl-thiophen-2-yl)-imidazo[1,2-b]pyridazin-6-yl]-pyridin-2-ylmethyl-amine;
(S)-3-Methyl-2-(3-pyrimidin-5-yl-imidazo[1,2-b]pyridazin-6-ylamino)-butan-1-ol;
4-[6-((S)-1-Hydroxymethyl-2-methyl-propylamino)-imidazo[1,2-b]pyridazin-3-yl]-2-methoxy-phenol;
4-[3-(3-Dimethylamino-phenyl)-imidazo[1,2-b]pyridazin-6-ylamino]-cyclohexanol;
Furan-2-ylmethyl-(3-pyrimidin-5-yl-imidazo[1,2-b]pyridazin-6-yl)-amine;
4-[6-(4-Hydroxy-cyclo hexylamino)-imidazo [1,2-b]pyridazin-3-yl]-benzonitrile;
3-[6-(4-Hydroxy-cyclohexylamino)-imidazo[1,2-b]pyridazin-3-yl]-benzoic acid;
4-[3-(4-Methoxy-phenyl)-imidazo[1,2-b]pyridazin-6-ylamino]-cyclohexanol;
4-(3-Thiophen-3-yl-imidazo[1,2-b]pyridazin-6-ylamino)-cyclohexanol;
4-[6-(4-Hydroxy-cyclohexylamino)-imidazo[1,2-b]pyridazin-3-yl]-benzoic acid;
4-[3-(4-Hydroxymethyl-phenyl)-imidazo[1,2-b]pyridazin-6-ylamino]-cyclohexanol;
4-[6-(4-Hydroxy-cyclohexylamino)-imidazo[1,2-b]pyridazin-3-yl]-2-methoxy-phenol;
1-{5-[6-(4-Hydroxy-cyclohexylamino)-imidazo[1,2-b]pyridazin-3-yl]-thiophen-2-yl}-ethanone;
4-(6-Propylamino-imidazo[1,2-b]pyridazin-3-yl)-phenol;
N,N-Dimethyl-N'-(3-thiophen-3-yl-imidazo[1,2-b]pyridazin-6-yl)-ethane-1,2-diamine;
4-[3-(2,4-Dimethoxy-pyrimidin-5-yl)-imidazo[1,2-b]pyridazin-6-ylamino]-cyclohexanol;
N-[3-(6-Propylamino-imidazo[1,2-b]pyridazin-3-yl)-phenyl]-acetamide;
N'-(3-Benzo[b]thiophen-2-yl-imidazo[1,2-b]pyridazin-6-yl)-N,N-dimethyl-ethane-1,2-diamine;
(2,4-Dimethyl-benzyl)-(3-thiophen-3-yl-imidazo[1,2-b]pyridazin-6-yl)-amine;
1-{5-[6-(3-Morpholin-4-yl-propylamino)-imidazo[1,2-b]pyridazm-3-yl]-thiophen-2-yl}-ethanone;
N'-[3-(3,4-Dimethoxy-phenyl)-imidazo[1,2-b]pyridazin-6-yl]-N,N-dimethyl-ethane-1,2-diamine;
(3-Chloro-benzyl)-(3-pyridin-4-yl-imidazo[1,2-b]pyridazin-6-yl)-amine;
(3-Benzo[1,3]dioxol-5-yl-imidazo[1,2-b]pyridazin-6-yl)-(3-morpholin-4-yl-propyl)-amine;
4-[3-(3-Hydroxymethyl-phenyl)-imidazo[1,2-b]pyridazin-6-ylamino]-cyclohexanol;
4-(3-Phenyl-imidazo[1,2-b]pyridazin-6-ylamino)-cyclohexanol;
(3-Morpholin-4-yl-propyl)-(3-thiophen-3-yl-imidazo[1,2-b]pyridazin-6-yl)-amine;
(2,4-Dimethyl-benzyl)-(3-pyridin-4-yl-imidazo[1,2-b]pyridazin-6-yl)-amine;
4-[6-(3-Morpholin-4-yl-propylamino)-imidazo[1,2-b]pyridazin-3-yl]-phenol;
4-[3-(3-Fluoro-phenyl)-imidazo[1,2-b]pyridazin-6-ylamino]-cyclohexanol;
N'-(3-Benzofuran-2-yl-imidazo[1,2-b]pyridazin-6-yl)-N,N-dimethyl-ethane-1,2-diamine;
3-(6-Propylamino-imidazo[1,2-b]pyridazin-3-yl)-benzamide;
[4-(6-Propylamino-imidazo[1,2-b]pyridazin-3-yl)-phenyl]-methanol;
Propyl-(3-pyrimidin-5-yl-imidazo[1,2-b]pyridazin-6-yl)-amine;
3-[6-(3-Morpholin-4-yl-propylamino)-imidazo[1,2-b]pyridazin-3-yl]-phenol;
2-Methoxy-4-(6-propylamino-imidazo[1,2-b]pyridazin-3-yl)-phenol;
1-{5-[5-(2-Hydroxy-ethylamino)-pyrazolo[1,5-a]pyrimidm-3-yl]-thiophen-2-yl}-ethanone;
N-{3-[5-(2-Hydroxy-ethylamino)-pyrazolo[1,5-a]pyrimidin-3-yl]-phenyl}-acetamide;
3-[6-(2-Dimethylamino-ethylamino)-imidazo[1,2-b]pyridazin-3-yl]-benzamide;
4-[3-(3-Methoxy-phenyl)-imidazo[1,2-b]pyridazin-6-ylamino]-cyclohexanol;
2-(3-Benzo[1,3]dioxol-5-yl-pyrazolo[1, 5-a]pyrimidin-5-ylamino)-ethanol;
4-{5-[(Pyridin-4-ylmethyl)-amino]-pyrazolo[1,5-a]pyrimidin-3-yl}-phenol;
4-(3-Pyridin-3-yl-pyrazolo[1,5-a]pyrimidin-5-ylamino)-cyclohexanol;
3-[5-(4-Acetylamino-phenylamino)-pyrazolo [1,5-a]pyrimidin-3-yl]-benzoic acid;
4-[3-(3,4-Dimethoxy-phenyl)-pyrazolo[1,5-a]pyrimidin-5-ylamino]-cyclohexanol;
2-[3-(3,4-Dimethoxy-phenyl)-pyrazolo[1,5-a]pyrimidin-5-ylamino]-ethanol;
4-[5-(4-Hydroxy-cyclohexylamino)-pyrazolo[1,5-a]pyrimidin-3-yl]-phenol;
(1H-Indol-5-yl)-[3-(5-methoxy-pyridin-3-yl)-pyrazolo [1,5-a]pyrimidin-5-yl]-amine;
4-[5-(2-Hydroxy-ethylamino)-pyrazolo [1,5-a]pyrimidin-3-yl]-benzonitrile;
2-[3-(3,4,5-Trimethoxy-phenyl)-pyrazolo [1,5-a]pyrimidin-5-ylamino]-ethanol;
2-Methoxy-4-[5-(3,4,5-trimethoxy-phenylamino)-pyrazolo [1,5-a]pyrimidin-3-yl]-phenol;
4-[3-(6-Methoxy-pyridin-3-yl)-pyrazolo [1,5-a]pyrimidin-5-ylamino]-phenol;
4-[5-(2-Dimethylamino-ethylamino)-pyrazolo[1,5-a]pyrimidin-3-yl]-benzoic acid;
[3-(4-Chloro-phenyl)-pyrazolo[1,5-a]pyrimidin-5-yl]-pyridin-3-ylmethyl-amine;
3-[5-(6-Methoxy-pyridin-3-ylamino)-pyrazolo [1,5-a]pyrimidin-3-yl]-phenol;
(6-Methoxy-pyridin-3-yl)-[3-(6-methoxy-pyridin-3-yl)-pyrazolo[1,5-a]pyrimidin-5-yl]-amine;
4-[3-(6-Methoxy-pyridin-3-yl)-pyrazolo[1,5-a]pyrimidin-5-ylamino]-cyclohexanol;
4-[5-(4-Hydroxy-cyclohexylamino)-pyrazolo [1,5-a]pyrimidin-3-yl]-2-methoxy-phenol;
3-{5-[(Pyridin-3-ylmethyl)-amino]-pyrazolo[1,5-a]pyrimidin-3-yl}-phenol;
(3-{5-[(Pyridin-3-ylmethyl)-amino]-pyrazolo[1,5-a]pyrimidin-3-yl}-phenyl)-methanol;
3-[5-(4-Hydroxy-cyclohexylamino)-pyrazolo[1,5-a]pyrimidin-3-yl]-phenol;
2-[3-(3-Hydroxymethyl-phenyl)-pyrazolo[1,5-a]pyrimidin-5-ylamino]-ethanol;
N'-[3-(4-Methoxy-phenyl)-pyrazolo[1,5-a]pyrimidin-5-yl]-N,N-dimethyl-ethane-1,2-diamine;
N-{5-[3-(4-Hydroxy-3-methoxy-phenyl)-pyrazolo[1,5-a]pyrimidin-5-ylamino]-2-methylphenyl}-methanesulfonamide;
(E)-3-{3-[5-(4-Hydroxy-cyclohexylamino)-pyrazolo[1,5-a]pyrimidin-3-yl]-phenyl}-acrylic acid;
2-[3-(6-Methoxy-pyridin-3-yl)-pyrazolo[1,5-a]pyrimidin-5-ylamino]-ethanol;
3-[5-(2-Dimethylamino-ethylamino)-pyrazolo [1,5-a]pyrimidin-3-yl]-phenol;
[3-(1-Methyl-1H-pyrazol-4-yl)-pyrazolo[1,5-a]pyrimidin-5-yl]-(3,4,5-trimethoxy-phenyl)-amine;
4-[3-(3-Hydroxymethyl-phenyl)-pyrazolo[1,5-a]pyrimidin-5-ylamino]-cyclohexanol;
4-[5-(2-Hydroxy-ethylamino)-pyrazolo[1,5-a]pyrimidin-3-yl]-2-methoxy-phenol;
3-[5-(3,4,5-Trimethoxy-phenylamino)-pyrazolo[1,5-a]pyrimidin-3-yl]-benzamide;
4-[3-(2-Hydroxymethyl-phenyl)-pyrazolo[1,5-a]pyrimidin-5-ylamino]-cyclohexanol;
3-[5-(4-Hydroxy-cyclohexylamino)-pyrazolo[1,5-a]pyrimidin-3-yl]-benzamide;
1-{3-[5-(4-Morpholin-4-yl-phenylamino)-pyrazolo[1,5-a]pyrimidin-3-yl]-phenyl}-ethanone;
Phenyl-(3-pyridin-4-yl-pyrazolo[1,5-a]pyrimidin-5-yl)-amine;
[3-(1-Methyl-1H-pyrazol-4-yl)-pyrazolo[1,5-a]pyrimidin-5-yl]-(4-morpholin-4-yl-phenyl)-amine;
3-{5-[(Pyridin-3-ylmethyl)-amino]-pyrazolo[1,5-a]pyrimidin-3-yl}-benzamide;
1-(5-{5-[(Pyridin-3-ylmethyl)-amino]-pyrazolo[1,5-a]pyrimidin-3-yl}-thiophen-2-yl)-ethanone;
N'-(3-Benzo[1,3]dioxol-5-yl-pyrazolo[1,5-a]pyrimidin-5-yl)-N,N-dimethyl-ethane-1,2-diamine;
N-(2-Dimethylamino-ethyl)-3-{5-[(pyridin-3-ylmethyl)-amino]-pyrazolo[1,5-a]pyrimidin-3-yl}-benzamide;
N-(2-Dimethylamino-ethyl)-4-{5-[(pyridin-3-ylmethyl)-amino]-pyrazolo[1,5-a]pyrimidin-3-yl}-benzamide;
4-{5-[(Pyridin-3-ylmethyl)-amino]-pyrazolo[1,5-a]pyrimidin-3-yl}-phenol;
N-(2-Dimethylamino-ethyl)-3-[5-(6-methoxy-pyridin-3-ylamino)-pyrazolo[1,5-a]pyrimidin-3-yl]-benzamide;
1-{5-[5-(2-Dimethylamino-ethylamino)-pyrazolo[1,5-a]pyrimidin-3-yl]-thiophen-2-yl}-ethanone;
4-[3-(3-Aminomethyl-phenyl)-pyrazolo[1,5-a]pyrimidin-5-ylamino]-phenol;
4-[3-(4-Fluoro-phenyl)-pyrazolo[1,5-a]pyrimidin-5-ylamino]-cyclohexanol;
[3-(3-Chloro-phenyl)-pyrazolo[1,5-a]pyrimidin-5-yl]-(4-morpholin-4-yl-phenyl)-amine;
[3-(3-Chloro-phenyl)-pyrazolo[1,5-a]pyrimidin-5-yl]-pyridin-3-ylmethyl-amine;
[3-(3-Amino-phenyl)-imidazo[1,2-b]pyridazin-6-yl]-(3-chloro-benzyl)-amine;
1-[3-(6-Propylamino-imidazo[1,2-b]pyridazin-3-yl)-phenyl]-ethanone;
[3-(4-Amino-phenyl)-imidazo [1,2-b]pyridazin-6-yl]-propyl-amine;
[3-(4-Chloro-phenyl)-imidazo [1,2-b]pyridazin-6-yl]-(3-morpholin-4-yl-propyl)-amine;
1-{3-[6-(3-Morpholin-4-yl-propylamino)-imidazo[1,2-b]pyridazin-3-yl]-phenyl}-ethanone;
N'-[3-(4-Fluoro-phenyl)-imidazo[1,2-b]pyridazin-6-yl]-N,N-dimethyl-ethane-1,2-diamine;
N,N-Dimethyl-N'-[3-(4-phenoxy-phenyl)-imidazo[1,2-b]pyridazin-6-yl]-ethane-1,2-diamine;
4-[6-(3-Chloro-benzylamino)-imidazo [1,2-b]pyridazin-3-yl]-N-(2-dimethylamino-ethyl)-benzamide;
N-(2-Dimethylamino-ethyl)-3-[6-(4-fluoro-benzylamino)-imidazo [1,2-b]pyridazin-3-yl]-benzamide;
[3-(3-Fluoro-phenyl)-imidazo[1,2-b]pyridazin-6-yl]-(3-morpholin-4-yl-propyl)-amine;
N'-[3-(2-Fluoro-phenyl)-imidazo[1,2-b]pyridazin-6-yl]-N,N-dimethyl-ethane-1,2-diamine;
N,N-Dimethyl-N'-(3-p-tolyl-imidazo[1,2-b]pyridazin-6-yl)-ethane-1,2-diamine;
4-(6-Propylamino-imidazo[1,2-b]pyridazin-3-yl)-benzoic acid;
N'-[3-(3-Chloro-phenyl)-imidazo [1,2-b]pyridazin-6-yl]-N,N-dimethyl-ethane-1,2-diamine;
1-{3-[6-(4-Fluoro-benzylamino)-imidazo[1,2-b]pyridazin-3-yl]-phenyl}-ethanone; and
1- {3-[6-(2-Pyridin-2-yl-ethylamino)-imidazo[1,2-b]pyridazin-3-yl]-phenyl}-ethanone.

27. A pharmaceutical composition comprising a compound or a pharmaceutically acceptable salt thereof according to any of the claims 1 to 26 together with a pharmaceutically acceptable carrier, optionally in combination with one or more other pharmaceutical compositions.

28. A pharmaceutical composition according to claim 27, comprising one or more additional compounds or pharmaceutically acceptable salts thereof selected from the group consisting of compounds according to any of the claims 1 to 26 and not being the first compound; other Itk inhibitors; steroids, leukotriene antagonsits, anti-histamines, cyclosporine or rapamycin.

29. A compound or a pharmaceutically acceptable salt thereof according to any of claims 1 to 26 for use as a medicament.

30. Use of a compound or a pharmaceutically acceptable salt thereof according to any of claims 1 to 26 for the manufacture of a medicament for the treatment or prophylaxis of diseases and disorders associated with Itk kinase.

31. Use of a compound or a pharmaceutically acceptable salt thereof according to any of claims 1 to 26 for the manufacture of a medicament for the treatment or prophylaxis of immunological, inflammatory or allergic disorders.

32. Use according to claim 31 for the manufacture of a medicament for the treatment or prophylaxis of autoimmune diseases, organ and bone marrow transplant rejection, graft-versus-host disease, acute or chronic inflammation, contact dermatitis, psoriasis, rheumatoid arthritis, multiple sclerosis, type I diabetes, inflammatory bowel disease, Crohn's disease, ulcerative colitis, graft versus host disease, lupus erythematosus, asthma, chronic obstructive pulmonary disease (COPD), acute respiratory distress syndrome (ARDS), bronchitis, conjunctivitis, dermatitis or allergic rhinitis.

33. A method for treating, controlling, delaying or preventing in a mammalian patient in need of treatment one or more conditions selected from the group consisting of immunological, inflammatory, allergic disorders and other diseases and disorders associated with Itk kinase, wherein the method comprises the administration to said patient an therapeutically effective amount of a compound according to any of claims 1 to 26 or a pharmaceutically acceptable salt thereof.

34. The method according to claim 33, wherein the one or more conditions are selected from the group consisting of autoimmune diseases, organ and bone marrow transplant rejection, graft-versus-host disease, acute or chronic inflammation, contact dermatitis, psoriasis, rheumatoid arthritis, multiple sclerosis, type I diabetes, inflammatory bowel disease, Crohn's disease, ulcerative colitis, graft versus host disease, lupus erythematosus, asthma, chronic obstructive pulmonary disease (COPD), acute respiratory distress syndrome (ARDS), bronchitis, conjunctivitis, dermatitis and allergic rhinitis.

35. A process for the preparation of a compound of formula (I) according to any of the claims 1 to 26, comprising the steps of
(a) reacting compounds of formula (II) 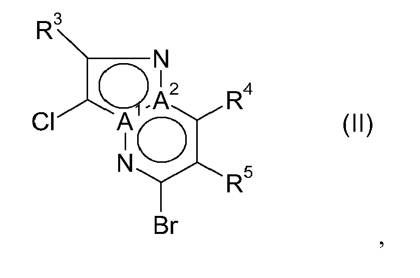 wherein A¹, A², R³ to R⁵ have the meaning as indicated in claim1 with compounds of the formula R²-XH, wherein R², X have the meaning as indicated in claim 1; and
(b) reacting the resulting intermediate from step (a) with boronic acid R¹-B(OH)₂, wherein R¹ has the meaning as indicated in claim 1, in a Suzuki reaction to yield compounds of formula (I).
